# EUROPEAN PATENT SPECIFICATION

(11) **EP 0 361 991 B1**
(45) Date of publication and mention of the grant of the patent: **15.12.1999**
(21) Application number: 89402217.7
(22) Date of filing: 04.08.1989
(51) Int. Cl.: C12P 21/02, C12N 15/81, C12N 15/62, C12N 15/14, C12N 1/19, A61K 38/00

(54) **Method for the preparation of human serum albumin from a yeast**
Verfahren zur Herstellung von menschlichem Serumalbumin in Hefe
Méthode de préparation du sérum d'albumine humaine à partir d'une levure

(30) Priority: 05.08.1988 FR 8810615; 03.07.1989 FR 8908897
(43) Date of publication of application: 04.04.1990
(73) Proprietor: RHONE-POULENC RORER S.A., 92160 Antony (FR)
(72) Inventor: Fleer, Rheinhard, F-91190 Gif sur Yvette (FR); Fukuhara, Hiroshi 160, Avenue du Général Leclerc, F-91190 Gif sur Yvette (FR); Yeh, Patrice, F-75005 Paris (FR)
(74) Representative: Ahner, Francis

(56) References cited:
- EP-A- 0 079 739
- EP-A- 0 241 435
- EP-A- 0 258 067
- EP-A- 0 301 670
- EP-A- 0 308 381
- EP-A- 0 319 641
- BIOTECHNOLOGY, vol. 4, no. 8, August 1986, New York, NY (US); T. ETCHEVERRY et al., pp. 726-730/
- JOURNAL OF BASIC MICROBIOLOGY, vol. 28, no. 4, 1988, Paper presented at the XIIth International Spezialized Symposium on Yeast: "Genetics of non-conventional yeasts", Weimar (DE), 13-19 September 1987; X.J. CHEN et al., pp. 211-220/
- CURRENT GENETICS, vol. 12, 1987, Springer-Verlag, Heidelberg (DE); M.M. BIANCHI et al., pp. 185-192/
- NUCLEIC ACIDS RESEARCH, vol. 14, no. 11, 1986, IRL Press Ltd., Oxford (GB); X.J. CHEN et al., pp. 4471-4481/
- BIOTECHNOLOGY, vol. 5, no. 12, December 1987, New York, NY (US); M. LATTA et al., pp. 1309-1314/
- BIOTECHNOLOGY AND GENETIC ENGINEERING REVIEWS, vol. 3, September 1985, Intercept Ltd., Newcastle-upon-Tyne (GB); S.M. KINGSMAN et al., pp. 377-416/

## Description

The present invention relates to a microbiological method for the preparation of human serum albumin (HSA) by culturing a yeast, especially of the genus Kluyveromyces, modified by the use of recombinant DNA techniques.

HSA is a protein of 585 amino acids with a molecular weight of 66 kilodaltons (kD), taking the form of a globular, unglycosylated monomer. Its globular structure is maintained by 17 disulfide bonds which create a sequential series of 9 double loops (Brown, J.R., in "Albumin Structure, Function and Uses", Rosenoer,V.M. et al. (eds.), Pergamon Press, Oxford, (1977) 27 - 51). The genes encoding HSA are known to be highly polymorphic, and more than 30 apparently different genetic variants have been typed by electrophoretic analysis under various conditions (Weitkamp, L.R. et al., Ann. Hum. Genet. 37 (1973) 219 - 226). The HSA gene is split into 15 exons by 14 intron sequences, and comprises more than 16.9 kilobases (kb) from the putative "Cap" site to the first poly(A) addition site.

Human albumin is synthesized in the liver hepatocytes, from which it is secreted into the blood-stream. It is the most abundant protein in the blood, with a concentration of about 40 g/liter of serum; there are hence about 160 g of circulating albumin in the human body at any time. The most important role of HSA is to maintain normal osmolarity in the bloodstream. It also has an exceptional binding capacity for various substances and plays, in addition, a role in the endogenous transport of hydrophobic molecules (e.g. steroids and biliary salts) as well as of various therapeutic substances which can thus be transported to their respective action sites. Furthermore, HSA has recently been implicated in the catabolism of prostaglandins.

The synthesis of HSA in the hepatocytes first yields a precursor, prepro-HSA, which contains a signal sequence of 18 amino acids directing the nascent polypeptide to the secretory pathway. This signal sequence is cleaved off, probably by cotranslational processing, before the protein is released from the endoplasmic reticulum. This first proteolytic cleavage yields the precursor pro-HSA, which still contains a N-terminal hexapeptide (Arg-Gly-Val-Phe-Arg-Arg) not normally present in the mature form of circulating HSA.

A convertase, probably located in the Golgi vesides removes the pro-peptide in a second proteolytic step by cleaving the hexapeptide bound to the N-terminal aspartic acid of mature HSA (Judah, J.D., and Quinn, P.L., Nature 271 (1987) 384 - 385; Bathurst, I.C. et al., Science 235 (1987) 348 - 350). However, non-processed human albumin may represent half of that present in the bloodstream in rare heterozygous individuals who carry an inherited mutation in the pro-peptide sequence, and all of the albumin in some extremely rare homozygotes (Takahashi, N. et al., Proc.Natl. Acad. Sci. USA 84 (1987) 7403 - 7407). One of these variants is designated pro-albumin Christchurch and begins with the sequence Arg-Gly-Val-Phe-Arg-Gln⁻¹-Asp⁺¹, in which the substitution Arg⁻¹→ Gln prevents the cleavage of the pro-peptide thus mutated (Brennan, S.O. and Carrel, R.W., Nature 274 (1978) 908 - 909). Other variants known as pro-albumin Lille (Abdo, Y. et al., FEBS Letters 131 (1981) 286 - 288) and Takefu (Nagata et al., Rinsho Byori 30 (1982) 791 - 796) have the following N-terminal sequences, respectively: Arg-Gly-Val-Phe-Arg-**His**-Asp and Arg-Gly-Val-Phe-Arg-**Pro**-Asp. In each of these cases, the observed mutation affects the pair of basic amino acids Arg-Arg which normally precedes the amino-acid sequence of mature human albumin, and results from a single-base change in the arginine codon (Takahashi, N. et al., Proc.Natl. Acad. Sci. USA 84 (1987) 7403 - 7407).

Cleavage after a pair of basic amino acids is an essential feature of the maturation of other proteins, including peptide hormones, neuropeptides and plasma proteins other than HSA (Douglass, O. et al., Annu. Rev. Biochem. 53 (1984) 665 pp.). Recently, it has been disclosed that the convertase which cleaves pro-albumin to albumin in the liver is probably very closely related to the protease yscF of yeast (Bathurst, I.C. et al., Science 235 (1987) 348 - 350). This calcium-dependent thiol protease is encoded by the KEX2 gene of Saccharomyces cerevisiae, and is probably bound to the membrane of the Golgi apparatus. It is known to be involved in the maturation of the pheromone referred to as alpha-factor and in that of the killer toxin, since kex2 mutants of S. cerevisiae are incapable of cleaving the respective pro-proteins at the pair of basic amino acids Lys-Arg (Julius, D.J. et al., Cell 32 (1983) 839 pp.). Furthermore, it can be demonstrated that the yeast enzyme encoded by the KEX2 gene correctly recognizes and cleaves the normal pro-sequence (Arg-**Arg**) of albumin in vitro, but does not cleave the mutated pro-sequence (Arg-**Gln**) of pro-albumin Christchurch (Bathurst, I.C. et al., Science 235 (1987) 348 - 350). A convertase analogous to the yscF protease has recently been described as being encoded by the KEX1 gene of K. lactis (Wésolowski-Louvel, M. et al., Yeast 4 (1988) 71 - 81). In the light of this finding, the capacity of these yeasts to maturate recombinant human albumin correctly and to secrete the latter into the culture medium was tested.

Albumin accounts for 40% of the world market in plasma proteins. Commercial interest in it stems from the fact that this product is widely used, for example, in so-called blood-volume expanders to compensate for blood losses during surgery, accidents, hemorrhages, and at doses per day and per individual in the multi-gram range.

Up to now, HSA is generally produced by conventional techniques of fractionation of plasma derived from blood donors (Cohn, E.J. et al., J. Am. Chem. Soc. 68 (1946) 459 pp), the worldwide annual consumption of plasma being in the region of 9.5 x 10⁶ liters. It can also be extracted from human placenta according to the technique described by J. Liautaud et al. (13th Intern. Congress of IABS, Budapest; A: "Purification of proteins. Development of biological standard", Karger (ed.), Bale, 27 (1973) 107 pp.).

The development of genetic engineering and of new extraction and purification techniques has opened up the possibility of obtaining improved products of higher purity and better stability without viral contamination (e.g. hepatitis B and AIDS) and at lower cost. However, no procedure based on genetic engineering is known as yet which is efficient enough to be of economic interest for the industrial-scale production of HSA.

This is mainly due to the lack of an efficacious host/vector system which would permit the high-level production of a correctly maturated secreted albumin possessing proper tertiary structure and having the physicochemical properties of native human albumin.

Even though the use of mammalian cell cultures might appear the ideal choice for the expression of human proteins, the cost of such a procedure would be well beyond the current selling price of albumin used for pharmaceutical purposes (Klausner, A., Biotechnology 3 (1985) 119 - 125). Since this product is generally prescribed in multi-gram quantities and at a low unit price, biotechnological production of HSA seems to be feasible only with microbial fermentation systems.

Until recently, the vast majority of genetic engineering experiments have employed Escherichia coli as the host organism for the microbial production of heterologous proteins of economic importance. Even though this type of procedure has appeared to be satisfactory for a number of heterologous proteins, all attempts to produce HSA under economically acceptable conditions using this organism have been only partially successful, (EP-A-0 079 739).

One of the major drawbacks associated with the use of E coli. lies in the fact that this bacterium is, in most cases, incapable of excreting the heterologous proteins into the culture medium, and that these proteins accumulate in one of the cellular compartments. The desired protein must hence be separated from cellular matter, giving rise to complex and expensive procedures. Furthermore, E. coli produces endotoxins and pyrogens which can contaminate the proteins produced. For this reason, great care must be taken in the purification to remove residual endotoxins in the end product, especially when the latter is used as a medicinal product.

Secreted proteins often lose the property of folding correctly if they are synthesized and accumulated in the cytoplasm. In general, secretion is required in order to make possible the formation of disulfide bonds such as those present in HSA. The fact that HSA produced in E. coli is not secreted thus causes it to precipitate intracellularly in an insoluble form (Latta, M. et al., Bio/Technology 5 (1987) 1309 - 1314). Consequently, after extraction from the bacterial cells, the protein has to be denatured and then renatured in vitro.

Furthermore, E. coli lacks the cellular machinery making it possible to perform the post-transcriptional and post-translational modifications specific to eukaryotic organisms, induding yeasts and humans. With regard to HSA, it appears to be difficult to induce E. coli to express at a satisfactory level the natural HSA precursor, i.e. prepro-HSA. Moreover, artificial albumins in which the HSA structural gene has been fused to secretion signals derived from the bacterial pac or ompA genes are generally processed incompletely (ibid.). Furthermore, the expression of HSA without the prepro-sequence, i.e. in the form Met-HSA, shows that the N-terminal methionine residue is not excised by the E. coli. methionine aminopeptidase (MAP) (ibid.; European patent application EP 198 745, publ. 22.10.1986). In consequence, E. coli-derived HSA cannot be obtained in mature form in vivo and must be modified in vitro, by tryptic cleavage, in order to excise a phage-derived leader sequence, subsequent to denaturation and renaturation in vitro (European patent application EP 236 210, publ. 09.09.1987).

As regards other bacterial hosts, studies have recently been published on the secretion of HSA by Bacillus subtilis (Saunders, C.W. et al., J. Bacteriol. 169 (1987) 2917 - 2925). Although this study indicates that a prokaryotic organism such as B. subtilis possesses the potential for secreting high molecular weight human proteins such as HSA, it also shows that excretion in the growth medium can be obtained only when using B. subtilis protoplasts, i.e. bacterial cells whose normal cell wall has been digested by enzymatic treatment. Furthermore, the percentage of modified HSA is inversely proportional to the level of protein produced: at high expression levels, most of the recombinant HSA remains in unmaturated form (ibid.). Moreover, no data concerning the physicochemical properties of the recombinant HSA from B. subtilis have been reported. Furthermore, the level of HSA excretion obtained by this microorganism is very low in the light of the amounts of HSA detected in the culture supernatants using immunological methods (European patent application EP 0 229 712 A2, publ. 22.07.1987).

An attractive alternative to the use of bacterial hosts for the preparation of heterologous proteins is the use of microbial eukaryotic systems such as yeasts or fungi. In effect, these organisms possess all the features of structure and cellular organization found in more complex eukaryotic organisms such as mammalian cells. In particular, yeasts are capable of carrying out the post-transcriptional and post-translational modifications which are important for the activity of many proteins. In addition, yeasts are very common in industrial-scale production, can be grown to high cell densities, are not pathogenic, do not produce endotoxins and have been used in the food industry since ancient times. Finally, in contrast to mammalian cells, genetic manipulations with yeasts are easy to perform, and classical and molecular genetics have provided a large body of data.

The term yeast is frequently used to indicate Saccharomyces cerevisiae, or baker's yeast, which is one of the most common and best known species. It is understood hereinafter that the term yeast applies to other genera, and is not restricted to the species S. cerevisiae.

The expression of prepro-HSA under the control of the chelatin promoter, has been demonstrated in S. cerevisiae at maximal levels of about 1% of total proteins (Etcheverry, T. et al., Bio/Technology 4 (1986) 726 - 730). However, the material recognized by anti-HSA antibodies which is described in this study remains cell-associated, and therefore is not truely exported into the culture supernatant. In addition, no detailed characterization of the recombinant protein has been reported.

Production of HSA in brewer's yeast using a post-fermentation process during beer brewing has also been reported (European patent application EP 0 201 239, publ. 12.11.1986). Once again, no quantitative or qualitative data concerning the product obtained has been described. Furthermore, this process leads to the expression of Met-HSA, i.e. an allele of HSA starting with the amino acid methionine just upstream from the sequence of mature albumin. The absence of a signal sequence precludes the secretion and maturation of the recombinant HSA, and causes accumulation of an intracellular albumin whose tertiary structure has not been characterized. In addition, the absence of N-terminal methionine in the final product has not been demonstrated.

EP-A-0 258 067, EP-A-0 308 381 and EP-A-0 319 641 describe the production of human serum albumin (HSA) from S. cerevisiae, but EP-A-0 258 067 does not indicate that HSA is excreted in the medium.

However, the present invention describes the production of genetically engineered, modified Kluyveromyces strains which can be grown in culture for mass production and which are capable of efficiently producing and excreting HSA in its native conformation into the growth medium.

A preferred expression system involves yeasts of the genus Kluyveromyces as a host and uses vectors derived from the natural K. marxianus var. drosophilarum plasmid pKD1.

Moreover, research performed with the object of producing albumin has revealed the value and importance of certain pKD1 sequences in the efficacy and especially the stability of the expression plasmids incorporating them.

Yeasts of the genus Kluyveromyces and in particular K. marxianus (including the varieties lactis and marxianus which will hereinafter be referred to as K. lactis and K. fragilis, respectively) are important organisms. Journal of Basic Microbiology, 28, n° 4, 1988, page 211-220 describes several different vectors to express a "killer" toxin of Kluyveromyces lactis, but this toxin is the result of the expression of an homologous gene of this yeast. However, yeasts of the genus Kluyveromyces may have a considerable commercial interest in the biotechnology industry. K. lactis and K. fragilis are used, for example, for the commercial production of the enzyme lactase (β-galactosidase). These yeasts are capable of growth on whey, a major byproduct of the dairy industry. Several Kluyveromyces strains are used for the large-scale production of "single cell protein" (SCP) which play an important role in livestock feeds. Finally, organisms of the genus Kluyveromyces are mentioned on the so-called GRAS list (Generally Recognized As Safe), which represents an important factor for the production of pharmaceutical grade products.

Gene manipulation techniques in Kluyveromyces have only recently been developed. Three types of cloning vectors have been described for this organism:
i) integrating vectors containing sequences homologous to regions of the Kluyveromyces genome and which, after being introduced into the cells, are integrated in the Kluyveromyces chromosomes by in vivo recombination. Integration, a very rare event requiring the presence of an efficient selection marker, is obtained when these vectors do not contain sequences permitting autonomous replication in the cell. The advantage of this system is the stability of the transformed strains, i.e. the fact that they can be grown in a normal nutrient support without the need for a selection pressure for maintenance of the integrated sequences. The disadvantage is, however, that the integrated genes are only present in one or, at best, a small number of copies per cell. This low gene dosage often results in a low level of production of a heterologous protein.
ii) replicating vectors containing autonomously replicating sequences (ARS) derived from the chromosomal DNA of Kluyveromyces sp. (Das, S. and Hollenberg, C.P., Current Genetics 6 (1982) 123 - 128; International patent application WO 83/04050, publ. 24.11.1983; International patent application WO 83/04051, publ. 24.11.1983). Such vectors are of only moderate interest, since their segregation in mitotic cell division is very unhomogeneous, which results in their loss from the cells at a high frequency even when the latter are grown under selection pressure. Furthermore, these vectors do not allow the obtention of the secreted protein at an industrial level (U.S. Patent N°4,859,596).
iii) replicating vectors derived from naturally occurring yeast plasmids, either from the linear killer plasmid pGKl-1 (k1) isolated from K. lactis (de Louvencourt, L. et al., J. Bacteriol. 154 (1982) 737 - 742; European patent

application EP 0 095 986, publ. 07.12.1983), or from the circular plasmid pKD1 isolated from K. marxianus var. drosophilarum (European patent application EP 0 241 435 A2, publ. 14.10.1987). Vectors containing replication sequences derived from the linear killer plasmid are of no practical utility for mass production of heterologous proteins since a special nutrient medium is required for their maintenance and since they are lost in 40-99% of the cells in a given population after only 15 generations, even under selection pressure (European patent application EP 0 095 986, publ. 07.12.1983). The most efficient vector system for the transformation of the genus Kluyveromyces described to date is derived from the endogenous plasmid pKD1: constructions containing the entire sequence of pKD1 can be transfered with high frequency, are present in the cell in 70-100 copies and, most importantly, have a relatively high stability under non-selective conditions. Nevertheless, the usefulness of the vectors described in EP 0 241 435 remains limited to research applications inasmuch as industrial-scale fermentations require a stability of the plasmid for at least 40 generations. Even the most efficacious vector (P3) described in EP 0 241 435 is lost by about 70% of the cells in a given population after only six generations in a non-selective medium (European patent application EP 0 241 435 A2, publ. 14.10.1987). Even though it is technically feasible to maintain a selection pressure in a large-scale fermentation, the use of selective medium often results in a considerably lower cell density, and requires the use of well defined strains, rendering this approach less attractive and more expensive. Furthermore, EP 0 241 435 does not contain a single example of expression of a heterologous protein of commercial interest. In fact, the only "heterologous" gene shown to be expressed from vectors derived from pKD1 is the URA3 gene of the yeast S. cerevisiae. It hence remains to be proven that the introduction of a non-yeast gene, e.g. a gene of mammalian origin, into pKD1 and its overexpression in Kluyveromyces do not result in enhanced plasmid instability.

EP-A-0 301 370 relates to the production of human serum albumin by K. lactis with a plasmid comprising an autonomous replication sequence functional in E. coli and a second replication origin functional in the host cell. pKD1 vector is mentioned as being likely to provide with replication sequences usable in Kluyveromyces but no information is given concerning the essential characteristics of a "functional" pKD1.

An object of the present invention is the production of new expression vectors capable of transforming yeasts of the genus Kluyveromyces and possessing stability characteristics markedly superior to those described in Patent Application EP 0 241 435. It will be shown that the new constructions described in the present invention are maintained at high copy numbers in 85-90% of the cells after 50 generations of growth in non-selective medium. It is thus possible to produce heterologous proteins from stable multicopy vectors using industrial strains of the genus Kluyveromyces which have been in use for many years on account of their optimal growth properties and at high cell densities.

The present invention relates, in particular, to a method for a preparation of human serum albumin (HSA), in which method a yeast of the genus Kluyveromyces transformed with an expression plasmid comprising:
- functional genes A, B, and C: of plasmid pKD1,
- the inverted repeats of plasmid pKD1 (IR),
- the stability locus of pKD1,
- the origin of replication of pKD1 and an expression cassette containing a no-yeast DNA encoding the structural gene for human serum albumin under the control of sequences permitting its expression in said yeast,
- a selectable marker for the transformed yeast,
- and, optionally, an origin of replication and a selectable marker for Escherichia coli,
is cultured in a growth medium.

The high stability of the vectors described in the present invention has been achieved by exploiting fully the characteristics of plasmid pKD1. Vectors derived from pKD1 differ from all other known Kluyveromyces vectors in that they contain a specialized replication system responsible for stable plasmid maintenance at high copy number. In addition to an origin of replication this system comprises two inverted repeats, each 346 nucleotides in length and three open reading frames (genes A, B and C) which are integral parts of the plasmid (Chen, X.J. et al., Nucl. Acids Res. 14 (1986) 4471 - 4481). The retention of these replication and stability elements, genes A, B and C and inverted repeats (IR) results in vectors exhibiting a very high mitotic stability. By analogy with the most extensively studied system, that of the structurally related 2 µ plasmid of S. cerevisiae, the proteins encoded by two of these genes (B and C) are probably involved in plasmid partitioning during mitotic cell division, and might play a part in the negative regulation of gene A encoding a site-specific recombinase (FLP; Futcher, A.B., Yeast 4 (1988) 27 - 40). FLP-mediated recombination between the inverted repeats of 2 µ DNA has been shown to switch the plasmid from a regular mode of replication (one doubling of 2 µ plasmids per cell division) to a rolling circle type mode of replication (copy number amplification to about 50 copies per cell; ibid.). This change in the normal mode of replication is induced as soon as the plasmid copy number becomes too low to permit the production of sufficient quantities of gene B and C products which act as repressors of gene A encoding the FLP recombinase. By this mechanism, the copy number of 2 µ (and very probably of structurally similar plasmids such as pKD1) is maintained at high levels in an autoregulated manner and is independent of the presence of a selectable marker.

The vectors previously published in EP 0 241 435 either contain only a part of pKD1 (A15) or contain an interrupted gene A ( P1 and P3 in which the PstI cloning site is located within the coding sequence of gene A (European patent application EP 0 241 435 A2, publ. 14.10.1987), thereby destroying the autoregulated replication system which is one of the characteristics of the resident plasmid pKD1. In contrast, plasmid constructions derived from pKD1 described in the present invention respect the functional integrity of all the important open reading frames of pKD1. In consequence, the stability of the plasmids described below is considerably enhanced with respect to plasmids P1 and P3, and the copy number of the new vectors is maintained at a high level throughout the cell population.

In the present invention, the term HSA will be used to denote any serum albumin of human origin or any protein isolated from yeast cells and possessing the same amino acid sequence, tertiary structure and physicochemical properties as native HSA of human origin. Furthermore, HSA variants are understood to denote natural variants, as well as molecules having the same activity as HSA but from which, where appropriate, the domains not essential to the activity in question have been truncated.

The present invention relates to the creation by genetic engineering of new vectors enabling HSA to be produced in a readily purifiable form using yeast cells as a eukaryotic microbial host. A feature of the production method is the efficient excretion of mature, native HSA into the growth medium of said yeasts, thereby considerably facilitating the purification of the recombinant protein. Production of HSA is obtained by growing yeasts transformed with a recombinant plasmid comprising a transcription and translation initiation region which functions efficiently in the said host and an open reading frame encoding HSA preceded by a secretion signal directing the recombinant protein to the secretory pathway of said yeasts.

According to the present invention, the new constructions include expression cassettes containing the structural gene for HSA or one of its variants. The constructions are usually prepared in a stepwise manner employing the appropriate vectors until the elements essential to expression, secretion or plasmid maintenance have been combined to form a vector which can then to introduced into the defined host(s) so as to express and excrete the desired protein. The hosts employed are of eukaryotic origin, especially yeasts, more especially of the genera Saccharomyces or Kluyveromyces and preferably the species Kluyveromyces marxianus including all its varieties, especially K. marxianus var. lactis. Thus, according to the present invention, the constructions which are prepared and described are in the nature of examples in eukaryotic organisms of microbiological origin, but are preferably directed to Kluyveromyces.

The particular hosts to be employed will preferably be industrial strains which are stable, grow to high cell densities in appropriate media and have a high level of production.

Among the strains which can be transformed with pKD1-derived plasmids, strains of the species K. wickerhamii, K. waltii and, in particular, K. marxianus var. bulgaricus (K. bulgaricus), K. marxianus var. drosophilarum (K. drosophilarum), K. marxianus var. marxianus (K. fragilis) and K. marxianus var. lactis (K. lactis) should be mentioned more especially.

The HSA coding sequence may be obtained in a variety of ways, the simplest consisting in isolating messenger RNA from human liver and synthesizing copies thereof in the form of complementary DNA (cDNA). The cloned sequences may then be modified by different methods such as in vitro site-directed mutagenesis, primer elongation, restriction, insertion of adaptors or ligation with oligodeoxynucleotide linkers. The coding sequence may be adapted, for example, to the use of prefered codons in yeast in order to optimize the efficiency of protein synthesis (translation) in the said host.

At the N-terminus of the protein sequence, a signal peptide (pre-sequence) may be introduced so as to direct the nascent protein to the secretory pathway of the host cell. This pre-sequence may correspond to the natural N-terminal leader of the protein, in particular albumin, or it may be of another origin, e.g. it may be obtained from yeast genes such as those encoding the alpha pheromone or the killer toxin.

Furthermore, a pro-sequence encoding another peptide extension may be interposed between the secretion signal sequence and the coding sequence for mature albumin. This pro-sequence is normally joined to the coding sequence by a site for cleavage by a specific protease generally involving at least two basic amino acids, preferably Lys-Arg or Arg-Arg.

The expression cassette will comprise a transcription and translation initiation region joined to the 5' terminus of the coding sequence, so as to direct and regulate the transcription and translation of said sequence. The choice of these promoter regions may vary according to the particular host used. These sequences are generally chosen from promoters derived from yeast genes. Of special interest are certain promoter and/or terminator regions derived from glycolytic genes of Saccharomyces or Kluyveromyces type yeast, such as the genes encoding phosphoglycerate kinase (PGK), glyceraldehyde-3-phosphate dehydrogenase (GDP), enolases (ENO), alcohol dehydrogenases (ADH), or derived from other strongly expressed genes such as the lactase (LAC4), the acid phosphatase (PHO5), and the like. These control regions may be modified, e.g. by in vitro mutagenesis, by introduction of additional control elements or synthetic sequences or by deletions. For example, transcription-regulating elements, such as the so-called UAS (upstream activating sequences) originating from another promoter, e.g. that of the S. cerevisiae GAL10 or K. lactis LAC4 gene, may be used to construct hybrid promoters which enable the growth phase of a yeast culture to be separated from the phase of expression of a heterologous gene, depending on the carbon source chosen. A transcription and translation termination region which is functional in the intended host will be positioned at the 3' end of the coding sequence.

The expression cassette thus constructed will be fused to one or more markers enabling the transformed host to be selected. Preferred yeast markers are dominant markers, i.e. markers conferring resistance to antibiotics such as geneticin (G418) as is the case with the expression in said yeast of the aph(3')-I gene (aph) of the bacterial transposon Tn903, or any other toxic compound such as copper ions, inasmuch as dominant markers may be used without special host requirements. These resistance genes will be placed under the control of appropriate transcription and translation signals in the host in question. It is also possible to use markers complementing auxotrophies such as the yeast genes URA3 or LEU2.

The assembly consisting of the expression cassette and the selectable marker may be used either for transforming the host directly or it can be inserted into a replicative vector. In the case of a direct transformation, sequences homologous to regions present on a chromosome or on a resident plasmid are fused to this assembly. Said sequences will be located on each side of the expression cassette so as to induce an insertion at the homologous site by in vivo recombination. The expression cassette can also be combined with a replication system which is functional in the host in question. A preferred replication system for Kluyveromyces is derived from the plasmid pKD1, initially isolated from K. drosophilarum (Falcone, C. et al., Plasmid 15 (1986) 248 - 252; Chen, X.J. et al., Nucl. Acids Res. 14 (1986) 4471 - 4481.). A preferred replication system for Saccharomyces is derived from the yeast 2 µ plasmid. The expression plasmid can contain all or part of said replication systems or can combine elements derived from pKD1 as well as from the 2 µ plasmid. Preferred constructions are those which contain the entire sequence of the plasmid pKD1 when expression in Kluyveromyces is desired. More especially, preferred constructions are those in which the insertion site of the foreign sequences into pKD1 is localized in a 197 bp region lying between the SacI site (position 4714 of the B-form of pKD1; Chen, X.J. et al., Nucl. Acids Res. 14 (1986) 4471 - 4481) and MstII site (position 154 of the B form of pKD1; ibid.), or at one of these two sites, or alternatively at the SphI site of plasmid pKD1.

For greater convenience the plasmid can be a shuttle vector: as such, it may be transferred to a bacterial host such as E. coli, where it may be manipulated more readily than in yeast at each construction step. In this case, an origin of replication and a selectable marker functioning in the bacterial host are required. It is also possible to position restriction sites which are unique on the expression vector so that they flank the bacterial sequences. This enables the bacterial origin of replication to be eliminated by cleavage and the vector to be religated prior to transformation of the yeast cells, and this may result in a higher plasmid copy number and enhanced plasmid stability. Convenient sites such as 5'-GGCCNNNNNGGCC-3' (SfiI) or 5'-GCGGCCGC-3' (NotI) can, for example, be used inasmuch as they are extremely rare in yeast and generally absent from an expression plasmid. These sites may be introduced into the vector by oligodeoxynucleotide-directed mutagenesis or by adding specific oligodeoxynucleotide linkers or adaptors.

Once construction of the expression vector has been completed, the latter will be introduced into the desired host. Various protocols for transformation have been described in the case of yeasts (Sherman, F. et al., "Methods in Yeast Genetics", Cold Spring Harbor Laboratory, Cold Spring Harbor, N.Y., 1986). The transformants may then be grown in a suitable medium in order to produce the desired product.

If the desired product is excreted, it may be purified in various ways from the culture supernatant. It may be cryoprecipitated, or extracted using affinity chromatography, electrophoresis or other conventional techniques. The secreted protein may be recovered from a batch culture or a continuous culture.

The examples which follow are given without implied limitation, and show special embodiments of the invention and certain advantages.

### DESCRIPTION OF THE FIGURES

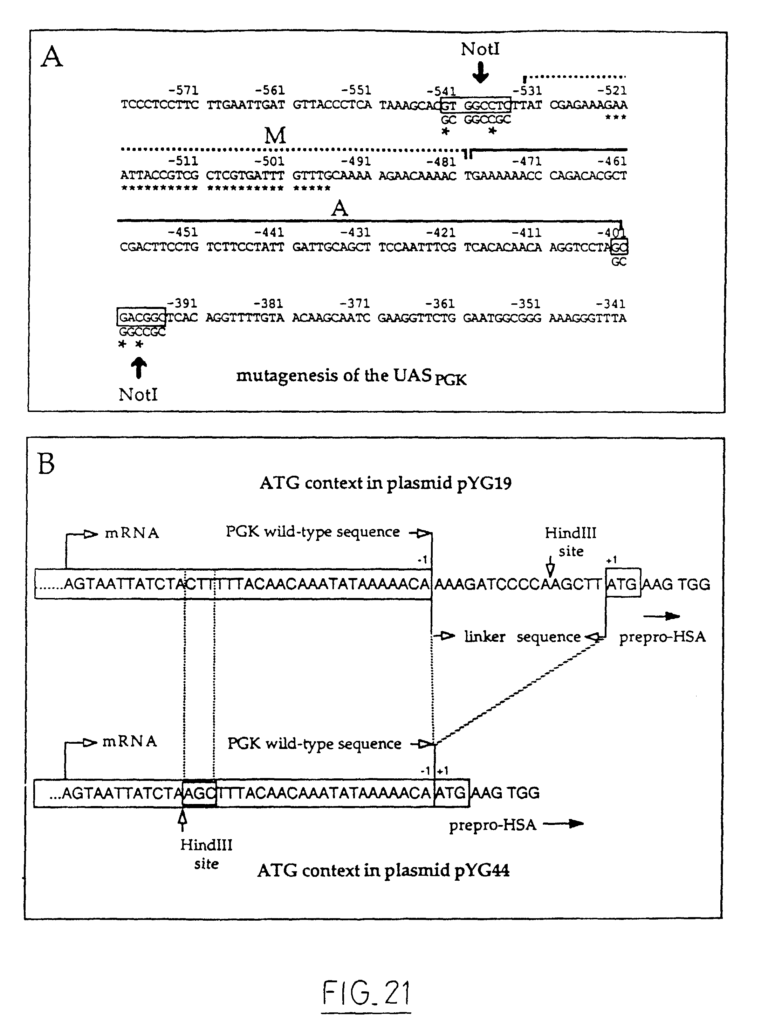

The diagrams of the plasmids shown in the figures are not drawn to scale, and only the restriction sites important for the constructions are shown.
- Fig. 1:: Construction of plasmid pXL276 containing the complete sequence encoding Met-HSA, obtained from three cDNA clones (see text) derived from poly(A)-mRNA isolated from human liver.
- Fig. 2:: Reconstitution of the prepro-sequence of albumin from four synthetic oligodeoxynucleotides : construction of plasmid pXL290.
- Fig. 3:: Construction of plasmid pXL299.
- Fig. 4:: Construction of plasmid pXL322.
- Fig. 5:: Construction of plasmid pXL855.
- Fig. 6:: Construction of plasmid pXL869.
- Fig. 7:: Nucleotide and amino acid sequence of the HindIII fragment derived from the plasmid pXL869 and containing the prepro-HSA structural gene. The heavy arrows indicate the end of the "pre" and "pro" regions.
- Fig. 8:: Construction of plasmid pUC-URA3.
- Fig. 9:: Construction of plasmid pCXJ1.
- Fig. 10:: Construction of plasmid pkl-PS1535-6.
- Fig. 11:: Construction of plasmid pUC-kan202.
- Fig. 12:: Nucleotide sequence of the ORF1 promoter of plasmid k1 from the ScaI restriction site and including the junction between ORF1 and the 5' region of the bacterial gene encoding aminoglycoside phosphotransferase derived from Tn903.
- Fig. 13:: Construction of plasmid pKan707.
- Fig. 14:: Stability curve of plasmid pKan707 in strain MW98-8C under non-selective growth conditions.
- Fig. 15:: Construction of plasmid pYG11.
- Fig. 16:: Construction of plasmid pYG18.
- Fig. 17:: Construction of plasmid pYG19.
- Fig. 18:: Illustration of plasmids pYG19 (prepro-HSA) and pYG23 (Met-HSA). The nucleotide sequence appearing under each plasmid is the junction between the PGK gene promoter and the structural genes for the different forms of HSA.
- Fig. 19:: Construction of plasmids pYG208, pYG210 (pannel A) and pYG221B (pannel B).
- Fig. 20:: Construction of plasmids pYG14, pYG26 and pYG29.
- Fig. 21:: **A**, Mutagenesis of the PGK promoter of S. cerevisiae to introduce NotI sites flanking the UAS region. The four base pairs changed by site directed mutagenesis are indicated by asterics below the NotI sites.
**B**, Untranslated PGK leader sequences in constructs pYG19 and pYG44. The HindIII site introduced at the -25 region of the wild-type PGK promoter by site directed mutagenesis is indicated by a box.
- Fig. 22:: Replacement of the UAS_{PGK} by a synthetic DNA fragment containing the UAS of the LAC4 promoter of K. lactis and reconstitution of the PGK wild-type ATG context via ligation of a synthetic adaptor to the mutagenized PGK promoter.
- Fig. 23:: SalI-SacI expression cassettes with various promoters and signal sequences present in pKan707 derived plasmids which direct the secretion of HSA into the growth medium.
- Fig. 24:: Site directed mutagenesis of the PHO5 promoter of S. cerevisae to introduce a HindIII site at position -20.
- Fig. 25:: Nucleotide sequence of the promoter region near the ATG initiator codon in construction pYG51.
- Fig. 26:: Reconstitution of a synthetic HindIII-DraI DNA fragment containing the 5' untranslated leader sequence of the PGK promoter, the signal sequence of the killer toxin of K. lactis (pre-region) and the first few codons of the pro-HSA gene.
Construction of plasmid pYG56.
- Fig. 27:: Schematic representation of plasmids p31/RAG2:URA3, pYG60-5 and pYG60-21.
- Fig. 28:: Mitotic stability of HSA expressing plasmids pYG19 and pYG23 under non-selective growth conditions: closed squares, strain MW98-8C transformed with plasmid pYG19; open squares, strain MW98-8C transformed with plasmid pYG23; open triangles, strain CBS683 transformed with plasmid pYG19.
- Fig. 29:: 8.5% SDS-polyacrylamide gel after staining with Coomassie Blue, demonstrating the expression and excretion of HSA from strain MW98-8C. Lanes 1-4: reference albumin extracted from human plasma (Sigma) spotted at increasing concentrations: 0.2 µg, 0.6 µg, 2 µg and 6 µg per lane. pYG19 (prepro-HSA), pYG23 (Met-HSA) and pYG25 (control vector without expression cassette): each lane corresponds to a quantity of protein equivalent to 100 µl of the original culture. a) soluble fractions; b) insoluble fractions; c) culture supernatants.

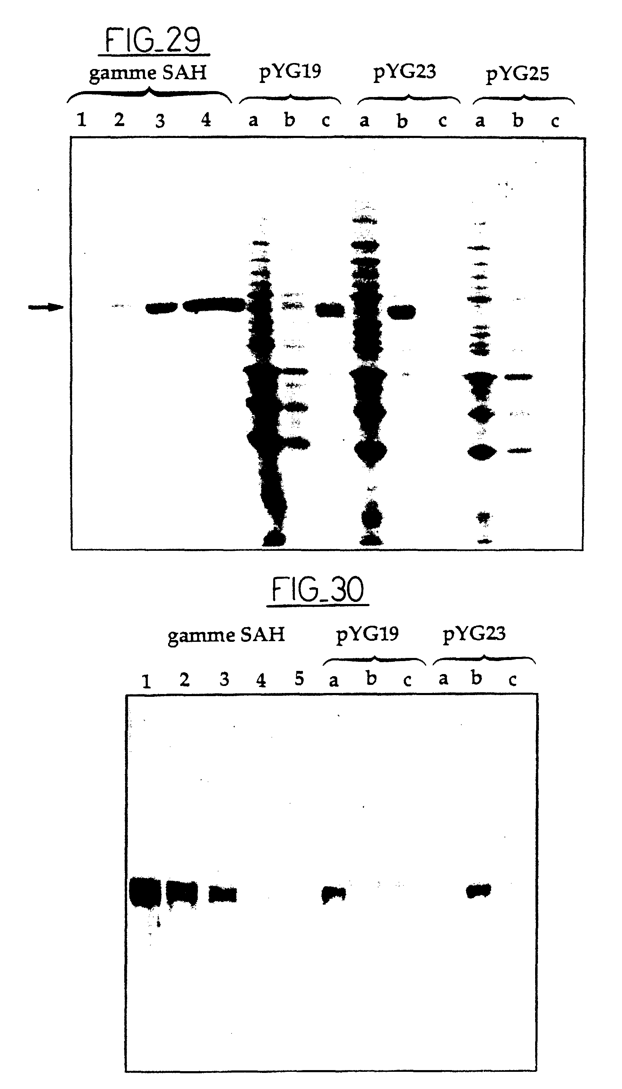

- Fig. 30:: Immunoblotting of a 7.5% polyacrylamide gel blotted onto nitrocellulose as described in the text. Lane 1-5: reference albumin extracted from human plasma (Sigma) which has been dissolved in a YPD medium at various concentrations and precipitated with TCA (5% final concentration) as described. The samples of the HSA standards have hence been treated in the same manner as the culture supernatants. In both cases, the gel spots correspond to equivalent volumes. 1) precipitation experiments with a concentration of HSA of 100 mg/l; 2) 50 mg/l; 3) 25 mg/l; 4) 12.5 mg/l; 5) 6 mg/l. pYG19 (prepro-HSA) and pYG23 (Met-HSA): each lane corresponds to a quantity of proteins equivalent to 20 µl of the original culture. a) culture supernatants; b) insoluble fractions; c) soluble fractions.
- Fig. 31:: 8.5% polyacrylamide gel after staining with Coomassie Blue, demonstrating the kinetics of excretion of HSA from strain MW98-8C.
**A**, lanes a-e: reference HSA (Sigma) which has been dissolved in YPD medium at various concentrations and precipitated with TCA (5% final concentration) as described (see legend of Figure 21). a) concentration of HSA (Sigma) of 6 mg/l; b) 12.5 mg/l; c) 25 mg/l; d) 50 mg/l; e) 100 mg/l. pYG19: pYG19-mediated secretion of HSA as a function of the age of the culture, 16-61 hours, as indicated. Each sample corresponds to the equivalent of 160 µl of culture supernatant.
**B**, lanes a-e: reference HSA (Sigma), spotted at increasing concentrations: 0.4 µg, 0.6 µg, 0.8 µg, 1.0 µg and 1.2 µg per lane. pYG19: pYG19-mediated secretion of HSA as a function of the age of the culture, 72-240 hours, as indicated. Each sample corresponds to the equivalent of 25 µl of non-concentrated culture supernatant.

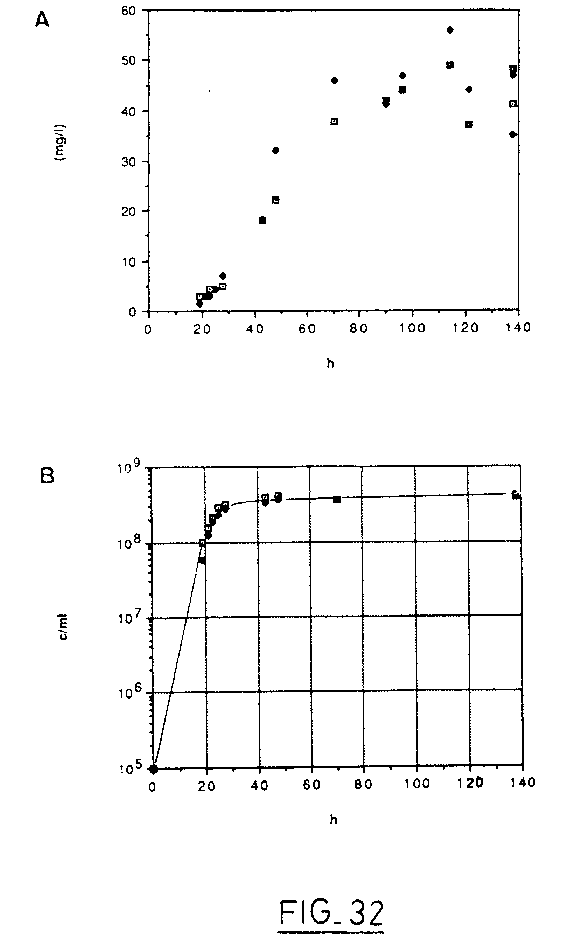

- Fig. 32:: **A**, graphic representation of the kinetics of excretion of HSA from strain MW98-8C transformed with plasmid pYG19 and grown in an Erlenmeyer as described in the text. The concentration of albumin detected in the growth medium (mg/l) is shown as a function of the age of the culture (hours). The results of four independent experiments are shown.
**B**, growth curve of strain MW98-8C transformed with plasmid pYG19.

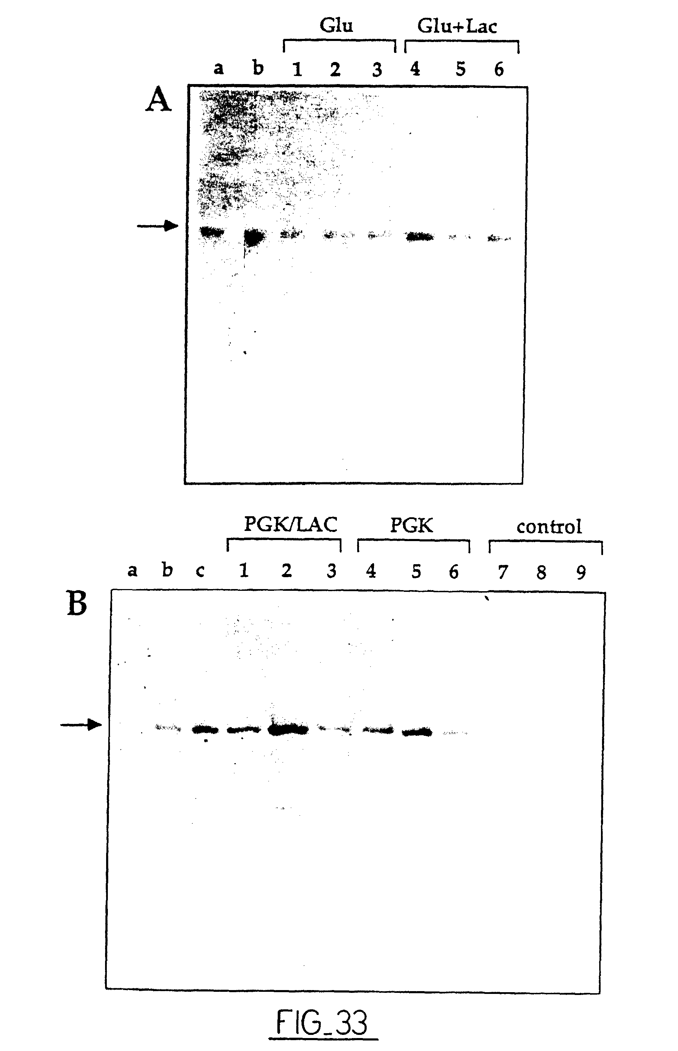

- Fig. 33:: Effect of carbon source on the efficiency of albumin secretion under control of the PGK or a PGK/LAC4 hybrid promoter: SDS-PAGE analysis of culture supernatants stained with Coomassie blue of K. lactis strain MW98-8C transformed with plasmids pYG19 and pYG44.
**A**, Supernatants (12.5 µl per lane) from shake-flask cultures grown for 211 h either in YPD (containing 2 % glucose) (lanes 1-3) or in YPD medium to which 2 % lactose has been added after 43 h of growth (lanes 4-6). Lanes a and b: HSA Sigma 0.5 and 1.0 µg, respectively; lanes 1 and 4: plasmid pYG44-5; lanes 2 and 5: plasmid pYG44-7; lanes 3 and 6: plasmid pYG19.
**B**, Supernatants (25 µl per lane) from shake flask cultures grown for 113 h either in YPD (containing 2 % glucose) (lanes 1, 4 and 7), YPL (containing 2 % lactose) (lanes 3, 6 and 9) or in TYD medium to which 2 % lactose has been added after 43 h of growth (lanes 2, 5 and 8). Lanes a, b and c: HSA Sigma 0.25, 0.5 and 1.0 µg, respectively; lanes 1-3: plasmid pYG44-5; lanes 4-6: plasmid pYG19; lanes 7-9: plasmid pKan707 without expression cassette.

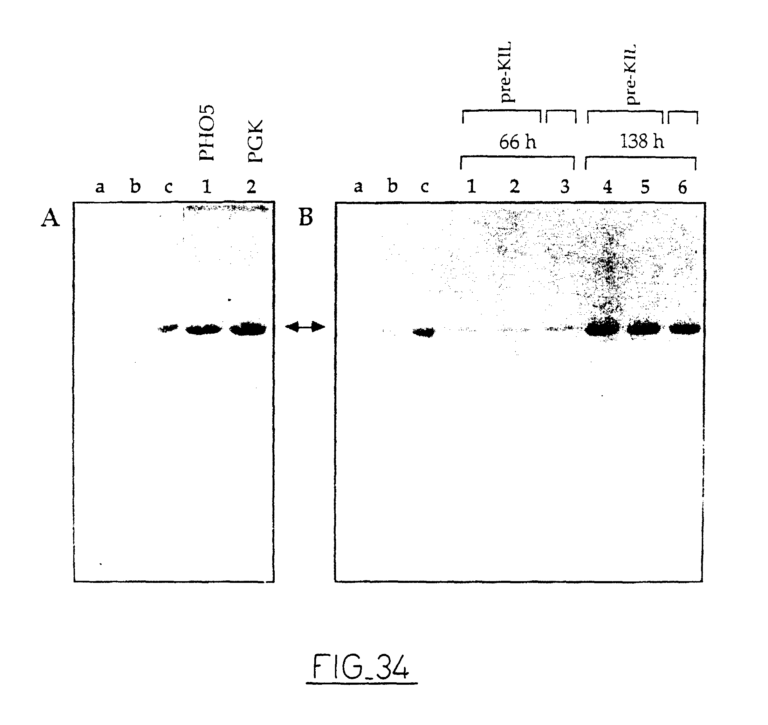

- Fig. 34:: Effect of promoter and signal sequence substitutions on the efficiency of albumin secretion: SDS-PAGE analysis of culture supernatants stained with Coomassie blue of K. lactis strain MW98-8C transformed with plasmids pYG51, pYG19 and pYG58.
**A**, Supernatants (25 µl per lane) from shake flask cultures grown for 115 h in YPD medium to which 2 % lactose has been added after 43 h of growth. Lanes a, b and c: HSA Sigma 0.2, 0.5 and 1.0 µg, respectively; lane 1; plasmid pYG51 (PHO5 promoter); lane 2: plasmid pYG19 (PGK promoter).
**B**, Supernatants (25 µl per lane) from shake flask cultures grown for 66 h (lanes 1-3) or 138 h (lanes 4-6) in YPD medium. Lanes a, b and c: HSA Sigma 0.25, 0.5 and 1.0 µg, respectively; lanes 1, 2, 4 and 5: plasmid pYG58 (killer toxin secretion signal, two independent transformants); lanes 3 and 6: plasmid pYG19 (albumin secretion signal).

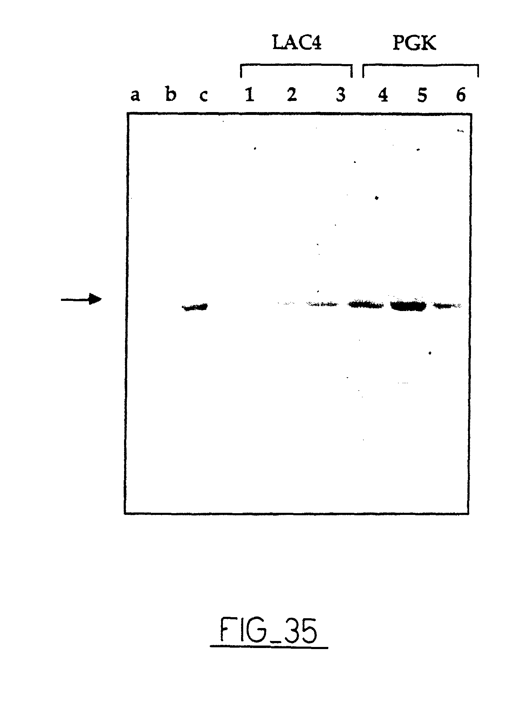

- Fig. 35:: Effect of carbon source on the efficiency of albumin secretion under control of the LAC4 or the PGK promoter: SDS-PAGE analysis of culture supernatants stained with Coomassie blue of K. lactis strain CBS2359 transformed with plasmids pYG404 and pYG19.
Supernatants (25 µl per lane) from shake flask cultures grown for 166 h either in YPD (containing 2 % glucose) (lanes 1 and 4), YPL (containing 2 % lactose) (lanes 3 and 6) or in YPD medium to which 2 % lactose has been added after 48 h of growth (lanes 2 and 5). Lanes a, b and c: HSA Sigma 0.25, 0.5 and 1.0 µg, respectively; lanes 1-3: plasmid pYG404; lanes 4-6: plasmid pYG19.

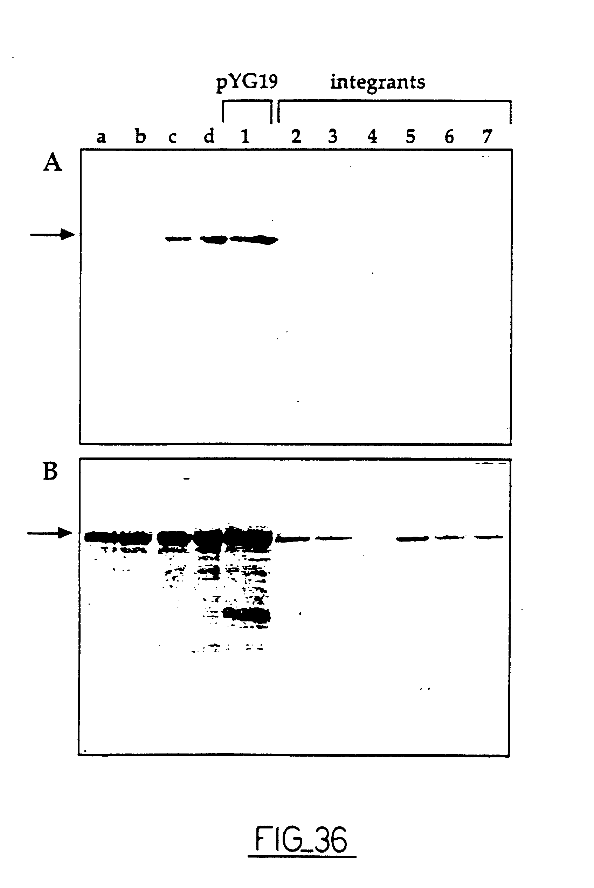

- Fig. 36:: Efficiency of albumin secretion under control of an integrated versus a plasmid based PGK expression cassette: SDS-PAGE and immunoblot analysis of culture supernatants of K. lactis integrant strains MW98-8C::60-5 and MW98-8C::60-21, and of strain MW98-8C transformed with plasmid pYG19. Supernatants (25 µl per lane) were sampled from shake flask cultures grown in YPD for 137 h.
**A**, PAGE-SDS: Lanes a, b, c and d: HSA Sigma 0.1, 0.25, 0.5 and 1.0 µg, respectively; lane 1: strain MW98-8C transformed with plasmid pYG19; lanes 2-7: 6 independent integrant strains (MW98-8C::60-5#6, MW98-8C::60-21#9, MW98-8C::60-21#7, MW98-8C::60-5#4, MW98-8C::60-5#8, MW98-8C::60-21#11, respectively; see section E.3.7).
**B**, Western blot analysis: same samples as shown in A (25 µl of supernantant deposited per lane).

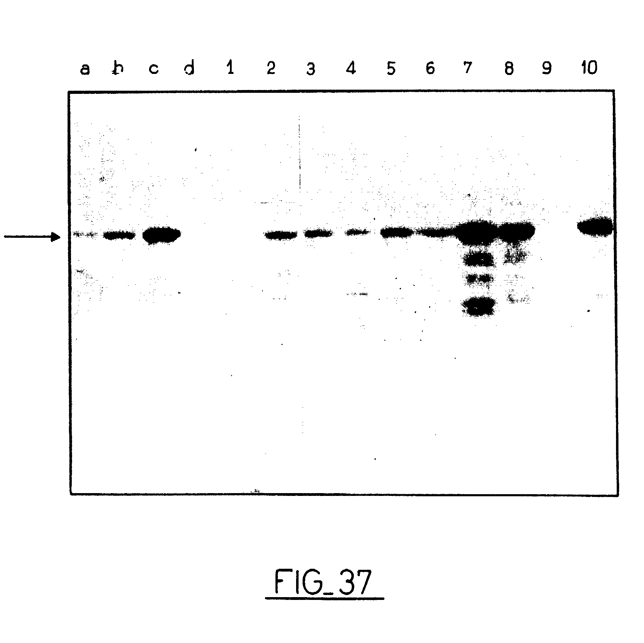

- Fig. 37:: Efficiency of albumin secretion in various Kluyveromyces strains transformed with plasmids pYG19 and pYG221B: SDS-PAGE analysis of concentrated culture supernatants stained with Coomassie blue. Cells were grown in YPD medium supplemented with G418 (200 µg/ml) for 89 h. Supernatants were concentrated ten-fold by microfiltration using centricon 30 microfiltration units (Amicon). The volume of concentrated supernatant deposited per lane was standardized as a function of biomass production and varies between 9 and 25 µl.
Lanes a and b: HSA Sigma 0.5 and 1.0 µg, respectively; lanes c and d: HSA Sigma subjected to microfiltration. A solution of HSA in YPD medium (10 mg/l) was concentrated ten-fold by microfiltration. 25 and 2.5 µl of the concentrated standard were deposited in lanes c and d, respectively. Lane 1: strain ATCC16045 (K. marxianus var. bulgaricus) transformed with plasmid pYG221B; lane 2: strain ATCC24178 (K. wickerhamii) transformed with plasmid pYG221B; lane 3: strain ATCC12424 (K. marxianus var. marxianus) transformed with plasmid pYG19; lane 4: strain ATCC56500 (K. waltii) transformed with plasmid pYG19; lane 5: strain ATCC36906 (K. marxianus var. drosophilarum) transformed with plasmid pYG221B; lane 6: strain CBS4574 (K. marxianus var. lactis) transformed with plasmid pYG19; lane 7: strain CBS683 (K. marxianus var. lactis) transformed with plasmid pYG19; lane 8: strain MW98-8C (K. marxianus var. lactis) transformed with plasmid pYG19; lane 9: strain MW98-8C (K. marxianus var. lactis) transformed with plasmid pKan707 (vector without expression cassette); lane 10: strain MW98-8C (K. marxianus var. lactis) transformed with plasmid pYG221B.

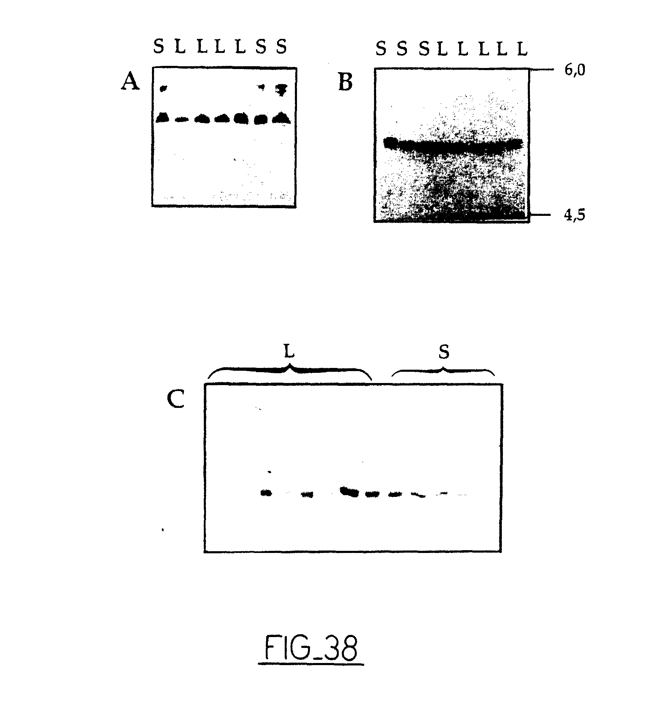

- Fig. 38:: Electrophoretic techniques: S = reference HSA (Sigma), L = yeast albumin.
**A** - PAGE-SDS (Phast gel, Pharmacia, gradient 8-25%) staining with silver salts, S = 1 µg, L: from 0.05 to 0.25 µg.
**B** - Isoelectric focusing (Phast gel, pH 4.5-6.0), staining with Coomassie Blue, spots = 1 µg.
**C** - PAGE SDS (7.5%) staining with Coomassie Blue.
   S = 0.2 - 1.0 µg, L = 0.25-2.5 µg.

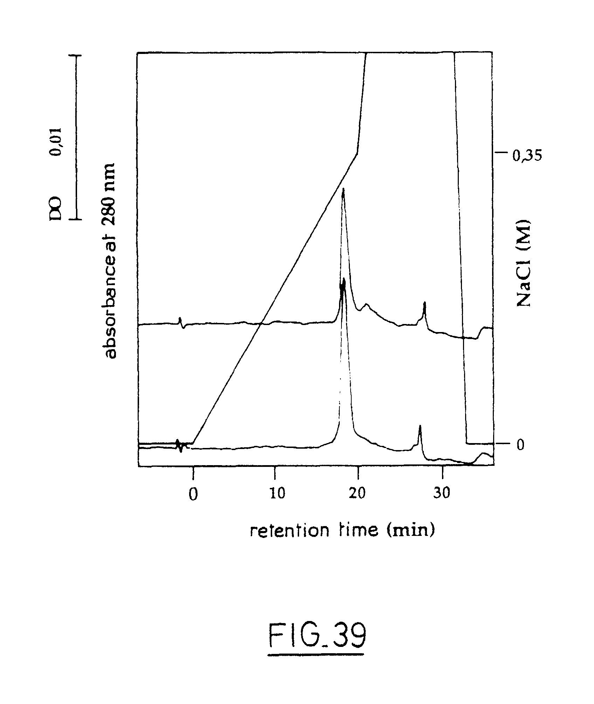

- Fig. 39:: Ion exchange chromatography - Mono Q (Pharmacia). Bottom: yeast HSA 40 µg; top: standard HSA 50 µg.

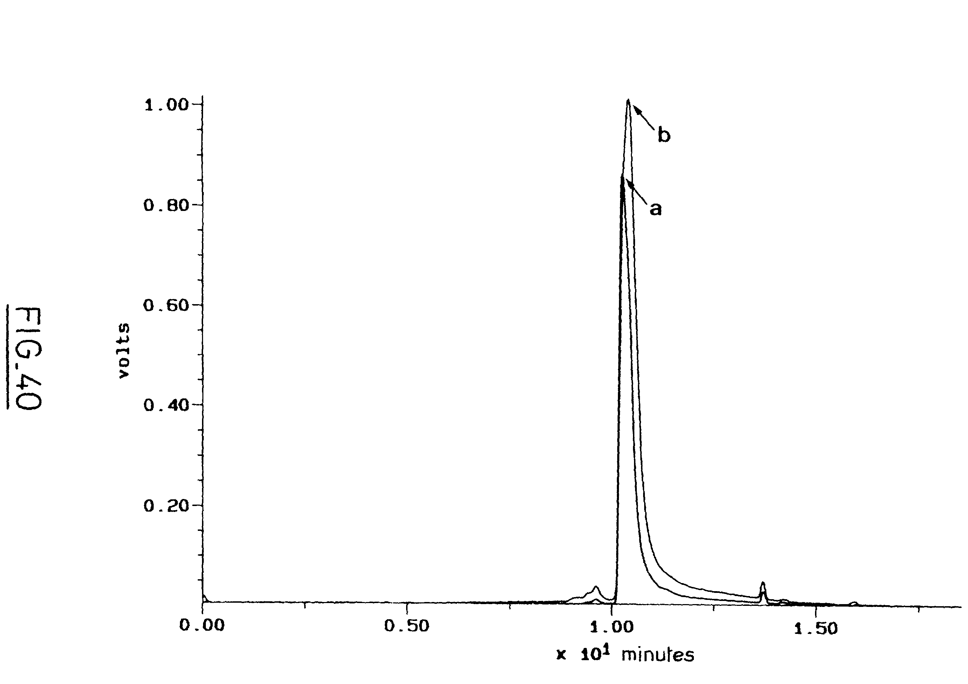

- Fig. 40:: Reversed-phase chromatography=Nucleosil C4. a) standard HSA; b) yeast HSA.

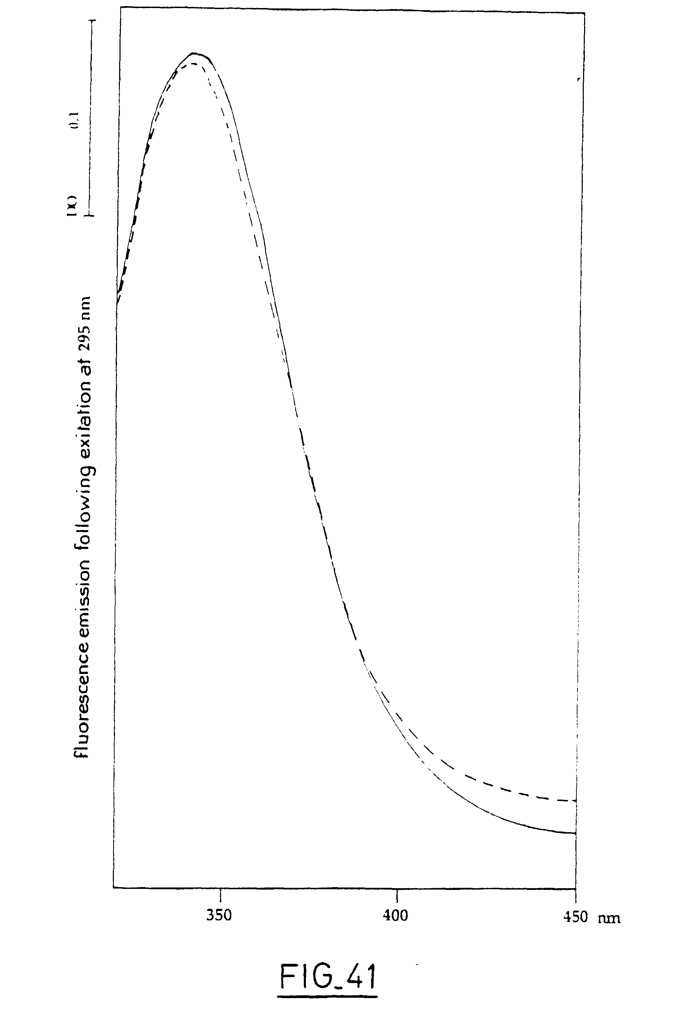

- Fig. 41:: Tryptophan fluorescence emission spectrometry. Solid line: standard HSA; broken line: yeast HSA.

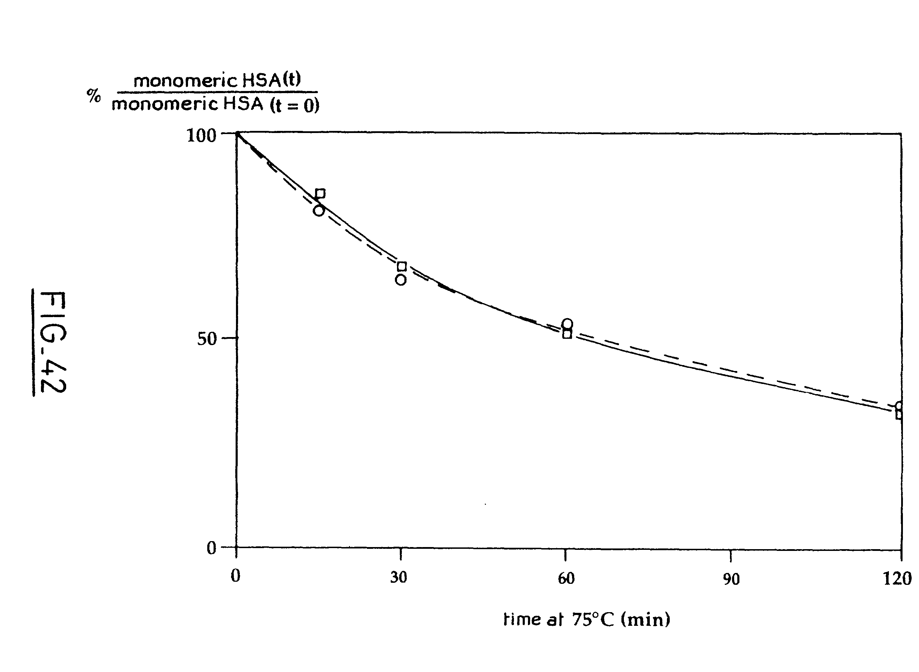

- Fig. 42:: Thermal stability at 75°C. Solid line: standard HSA; broken line: yeast HSA.

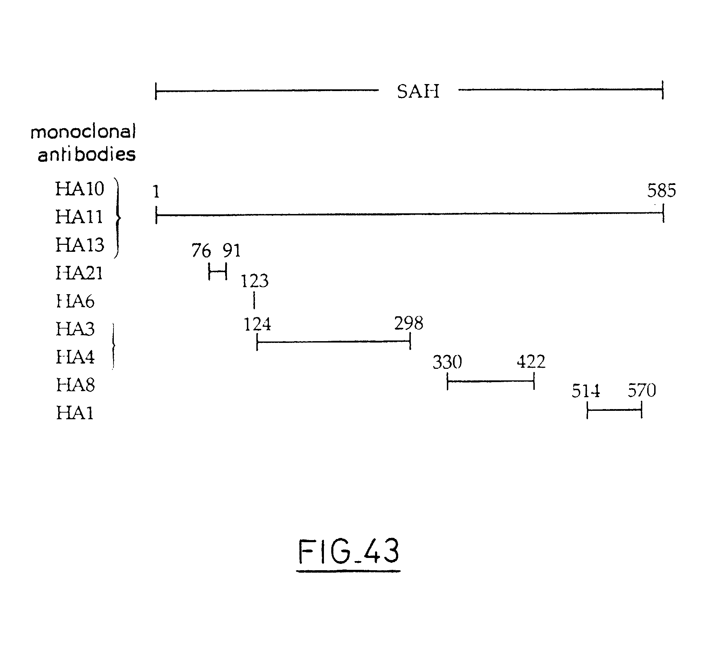

- Fig. 43:: Regions of the albumin recognized by the different monoclonal antibodies used for antigenic characterization.

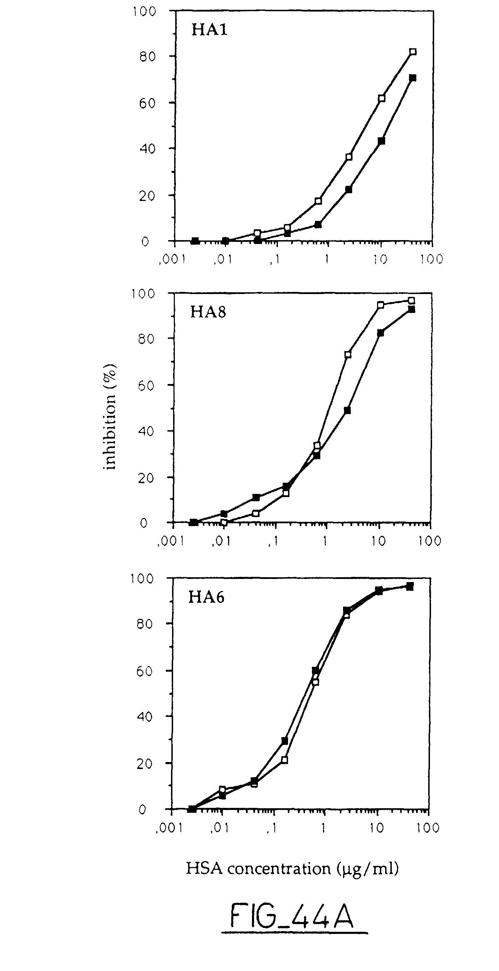

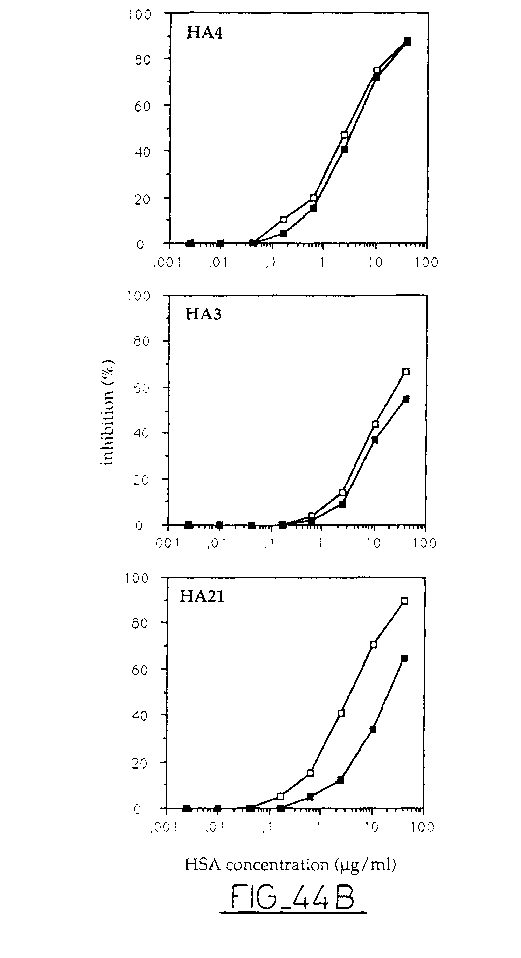

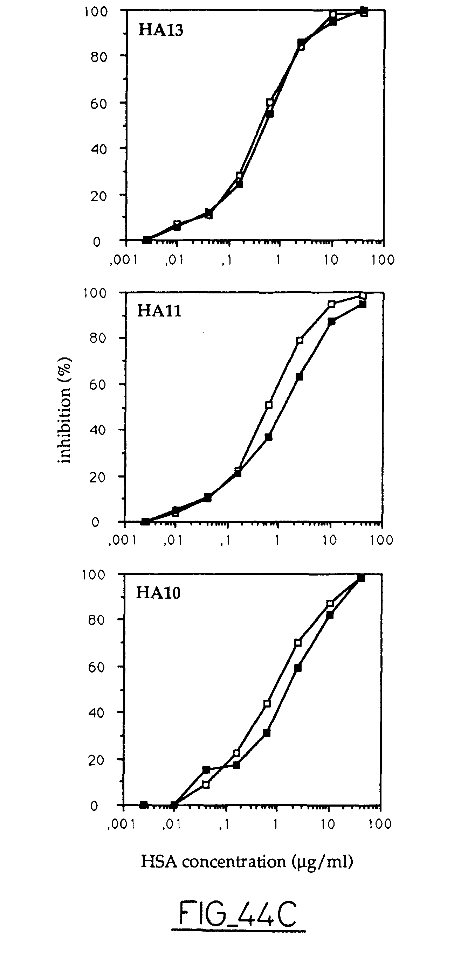

- Fig. 44:: **A,B,C**: inhibition curves (see text) with respect to the nine monoclonal antibodies whose specificity is shown in Figure 43: percentage inhibition as a function of the albumin concentration (µg/ml); open squares: standard HSA; filled squares: yeast HSA.

### EXAMPLES

### General cloning techniques

Classical methods of molecular biology such as cesium chloride-ethidium bromide gradient centrifugation of plasmid DNA, restriction enzyme digestion, gel electrophoresis, electroelution of DNA fragments from agarose gels, transformation in E. coli and the like, are described in the literature (Maniatis, T. et al., "Molecular Cloning: a Laboratory Manual", Cold Spring Harbor Laboratory, Cold Spring Harbor, N.Y., 1982; Ausubel, F.M. et al. (eds.), "Current Protocols in Molecular Biology", John Wiley & Sons, New York 1987).

The restriction enzymes were obtained from New England Biolabs (Biolabs), Bethesda Research Laboratories (BRL) or Amersham.

For ligation, DNA fragments are separated, according to size, on 0.7% agarose or 8% acrylamide gels, purified by electroelution, extracted with phenol, precipitated with ethanol and then incubated in a buffer comprising 50 mM Tris-HCL, 10 mM MgCl₂, 10 mM dithiothreitol, 2 mM ATP, pH 7.4, in the presence of phage T4 DNA ligase (Biolabs).

Where necessary, DNA fragments having 3' recessed termini are dephosphorylated by treatment with calf intestinal alkaline phosphatase (CIP, Pharmacia) at 37°C for 30 minutes in the following buffer: 100 mM glycine, 1 mM MgCl₂, 1 mM ZnCl₂, pH 10.5. The same procedure is used for dephosphorylation of 5' recessed or blunt-ended termini, but the treatment is for 15 minutes at 37°C and then 15 minutes at 56°C. The enzyme is inactivated by heating the reaction mixture to 68°C for 15 minutes in the presence of 1% SDS and 100 mM NaCl followed by extraction with phenol/chloroform and ethanol precipitation.

Filling in of the 3' recessed termini is performed with the Klenow fragment of E. coli DNA polymerase I (Biolabs). The reaction is performed at room temperature for 30 minutes in a buffer comprising 50 mM Tris-HCl, 0.4 mM dNTPs, 10 mM MgSO₄, 0.1 mM dithiothreitol, 50 µg/ml BSA (bovine serum albumin) pH 7.2. Blunt-ending of 5' recessed termini is performed in the presence of phage T4 DNA polymerase (Biolabs) as recommended by the manufacturer. Digestion of recessed ends is performed by limited treatment with S1 nuclease (BRL) as recommended by the manufacturer.

In vitro oligodeoxynucleotide-directed mutagenesis is performed according to the method developed by Taylor et al. (Nucleic Acids Res. 13(1985) 8749 - 8764), using the kit distributed by Amersham. Nucleotide sequencing is performed according to the dideoxy sequencing method (Sanger F. et al., Proc. Natl. Acad. Sci. USA, 74 (1977) 5463 - 5467). Enzymatic amplification of specific DNA fragments are performed by the Polymerase-catalyzed Chain Reaction procedure (Mullis K.B. and Faloona F.A., Meth. Enzym. 155 (1987) 335 - 350; Saiki R.K. et al., Science 230 (1985) 1350 - 1354) with a "DNA thermal cycler" (Perkin Elmer Cetus) following the recommendations of the supplier.

Ligated DNA is used for transforming competents cells obtained from the following strains: E. coli MC1060 ([lacIPOZYA], X74, galU, galK, strA^{r}) or TG1 ([lac pro A,B], supE, thi, hsd D5/F' traD36, proA⁺B⁺, lacI^{q}, lacZ M15). Plasmid DNA is purified from ampicillin- or tetracycline-resistant transformants as appropriate. Extraction of plasmid DNA is carried out according to the procedure described by Maniatis et al. (Maniatis, T. et al., "Molecular Cloning: a Laboratory Manual", Cold Spring Harbor Laboratory, Cold Spring Harbor, N.Y., 1982), which is derived from the alkaline lysis method described by Birnboim and Doly (Birnboim, H.C. and Doly, J., Nucleic Acids Res. 7 (1979) 1513 - 1523). For rapid plasmid analysis, bacterial lysates are prepared according to the method of Holmes and Quigley (Holmes, D.S. and Quigley, M., Anal. Biochem. 114 (1981) 193 - 197) and subjected to agarose gel electrophoresis analysis without prior purification. After endonuclease restriction analysis, recombinant plasmids exhibiting the desired structure are prepared on a larger scale according to the alkaline lysis procedure (Maniatis, T. et al. , "Molecular Cloning: a Laboratory Manual", Cold Spring Harbor Laboratory, Cold Spring Harbor, N.Y., 1982) using 0.5 to 1 liter cultures, and purified by cesium chloride density centrifugation.

Transformation of K. lactis with foreign DNA and purification of plasmid DNA from K. lactis are described in the text.

### Example 1: CLONING OF A CASSETTE CONTAINING THE PREPRO-HSA STRUCTURAL GENE

### E.1.1 Isolation of a complementary DNA (cDNA) clone encoding HSA.

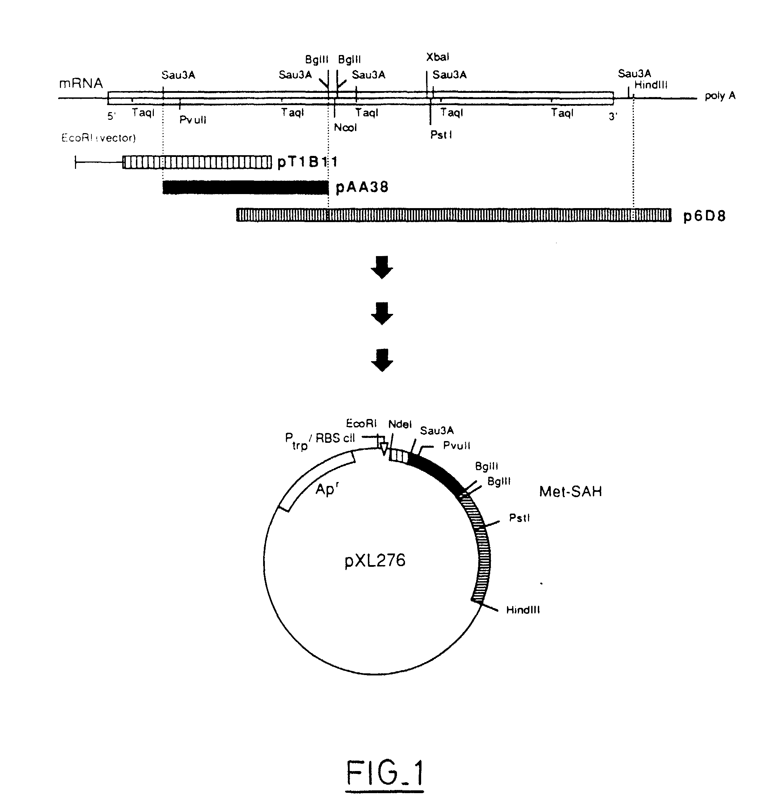

The construction of recombinant plasmids permitting the expression of HSA in E. coli has been described in detail in the previous Patent Application EP 0 198 745 (European patent application EP 198 745, publ. 22.10.1986). In summary, cDNA was obtained from poly(A)mRNA isolated from human liver according to the guanidine thiocyanate procedure (Chirgwin, J.M. et al., Biochemistry 18 (1979) 5294 pp.). DNA sequence analysis permitted the isolation of three clones (pT1B11, pAA38 and p6D8, Figure 1) which contained overlapping fragments of the albumin structural gene and possessed common restriction sites within these overlaps. These sites were used in the reconstruction of a cDNA clone for HSA (Figure 1) which is complete except for its prepro-sequence.

### E.1.2 Synthesis of the HSA prepro-sequence

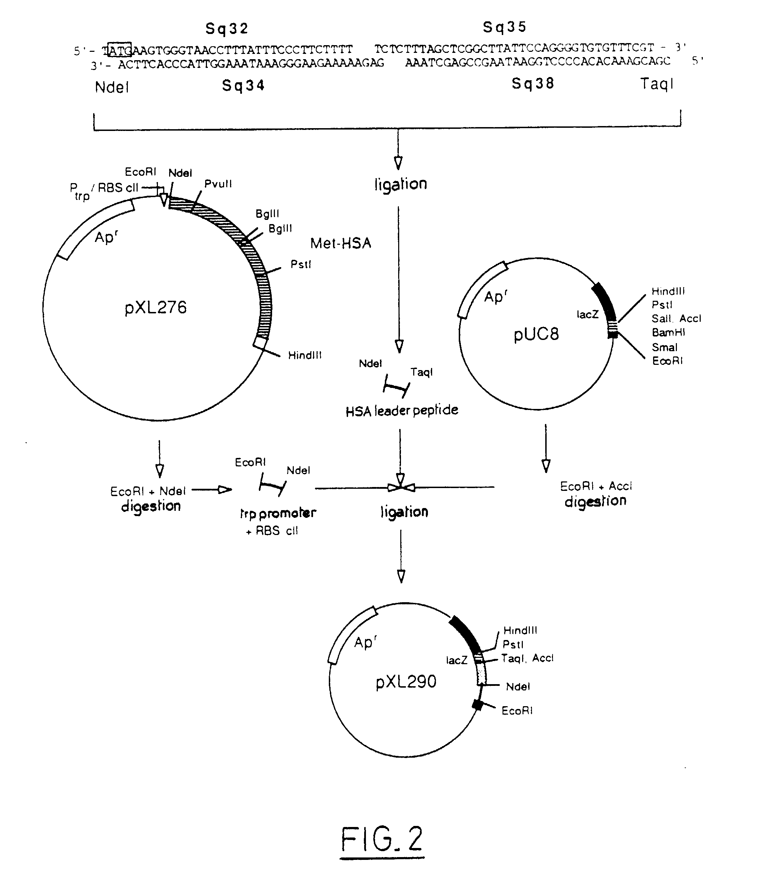

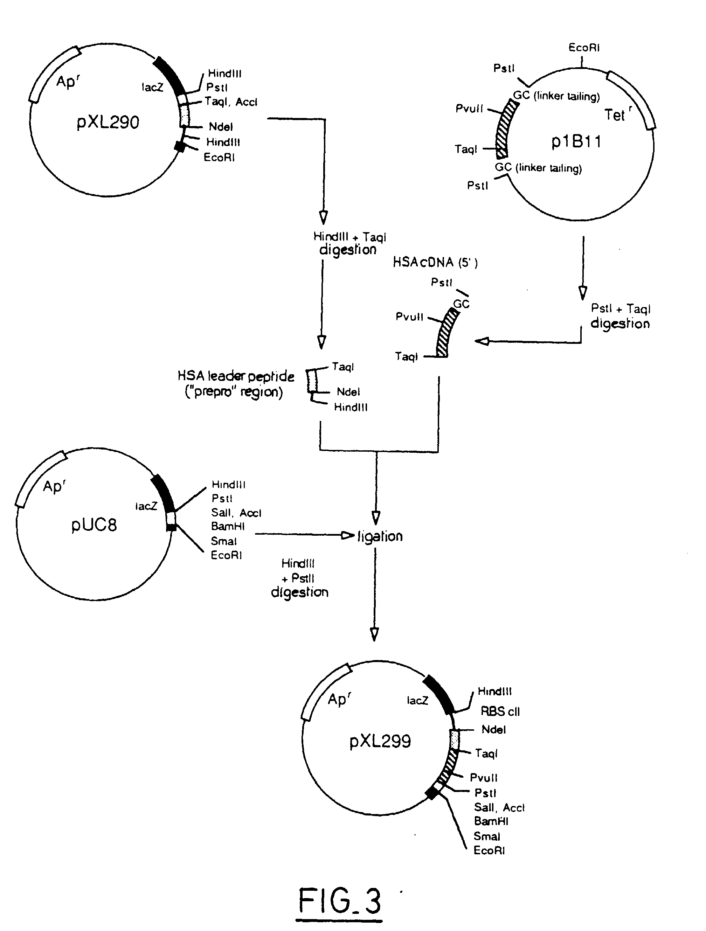

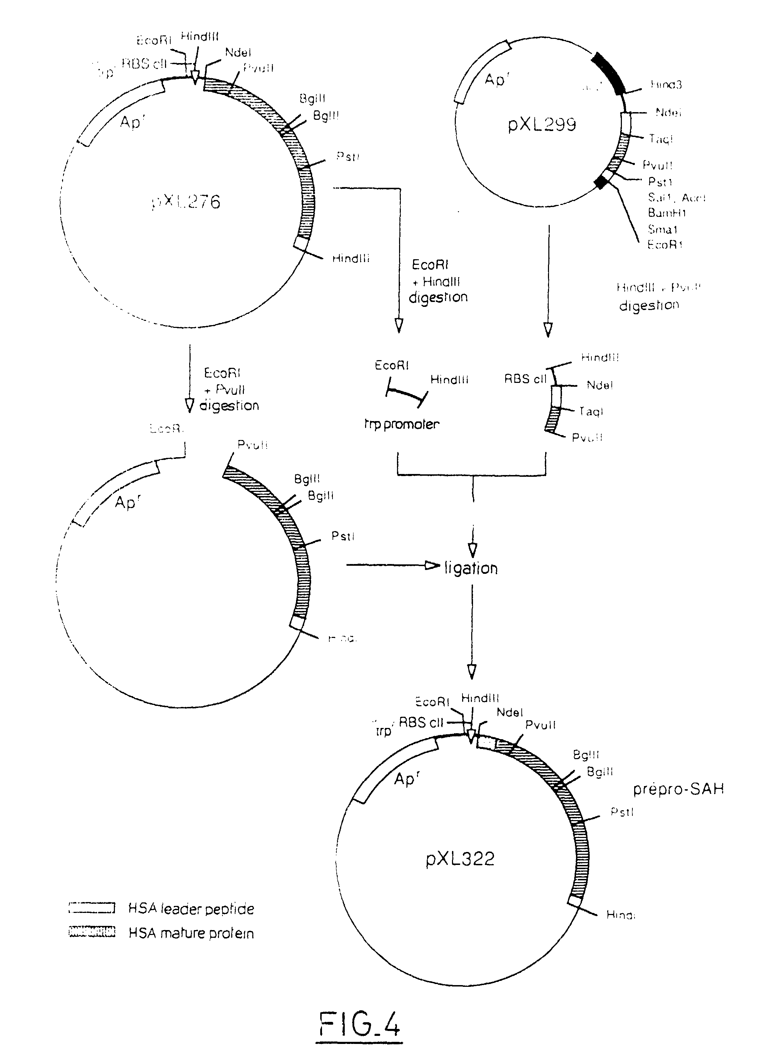

In plasmid pXL276, the construction of which is described in detail in Patent Application EP 0,198,745, the coding sequence for mature HSA has been fused in frame to the ATG codon of the ribosome binding site (RBS) of the cII gene of bacteriophage lambda (Figure 2). This generated an NdeI site immediately upstream from the translation initiation codon of the HSA gene. pXL276 is used for reconstituting the prepro-region of HSA, which is found to be truncated in the cDNA just upstream from the TaqI site at amino acid "-1". Reconstitution is achieved by inserting a DNA fragment corresponding to the HSA prepro-sequence in the form of four oligodeoxynucleotides 33-36 bases long (Sq32, Sq34, Sq35 and Sq38; Figure 2) and a 120 bp EcoRI-NdeI fragment derived from pXL276 carrying expression signals of E. coli genes between the EcoRI and AccI sites of plasmid pUC8. The TaqI site is thus reconstituted at one end of the insert in this plasmid designated pXL290 (Figure 2). The small HindIII-TaqI fragment carrying the RBS and the prepro-sequence upstream from the TaqI site is ligated with the TaqI-PstI fragment of the HSA cDNA clone (plasmid plB11, containing the 5' end of HSA) between the HindIII and PstI sites of pUC8 to give plasmid pXL299 (Figure 3). Plasmid pXL322 is constructed by ligating the HindIII-PvuII fragment of pXL299, carrying the RBS and the prepro-HSA sequence up to the PvuII site, with the large PvuII-EcoRI fragment of pXL276, bearing the replicon and the 3' end of the HSA coding sequence, and with the EcoRI-HindIII fragment of pXL276 containing the promoter (Figure 4).

### E.1.3 Creation of a HindIII site upstream from the HSA translation initiation codon

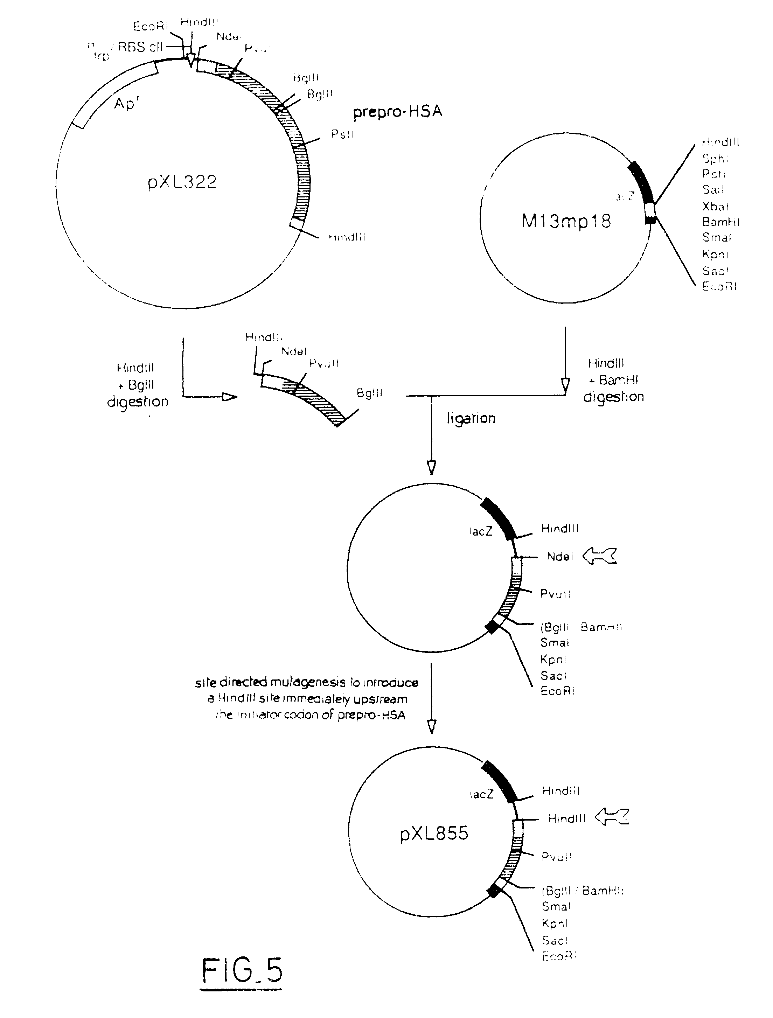

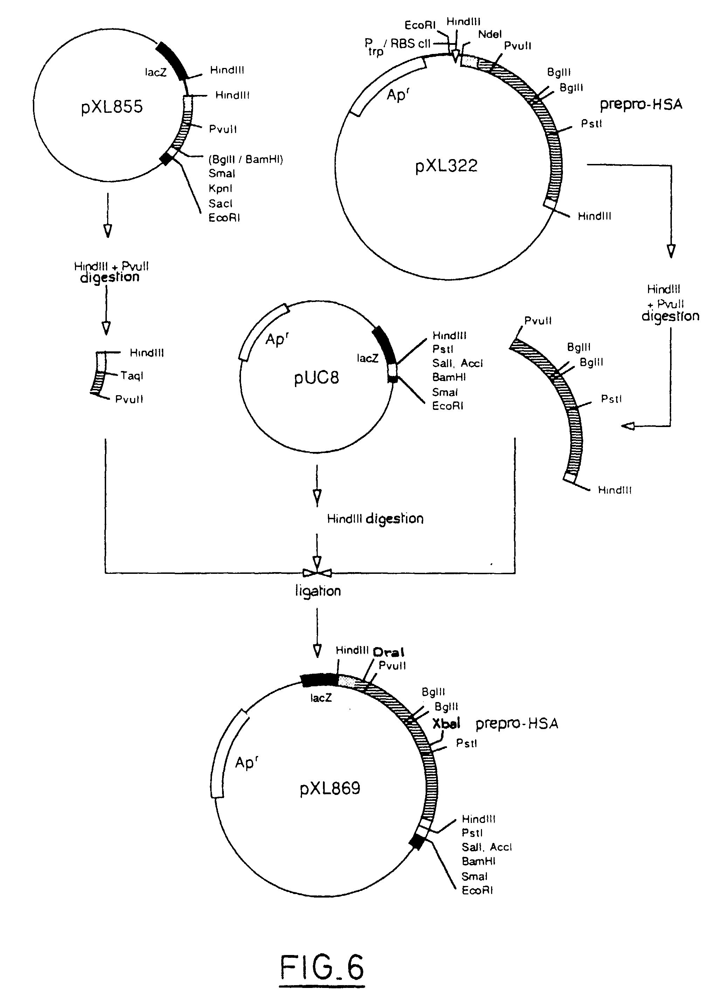

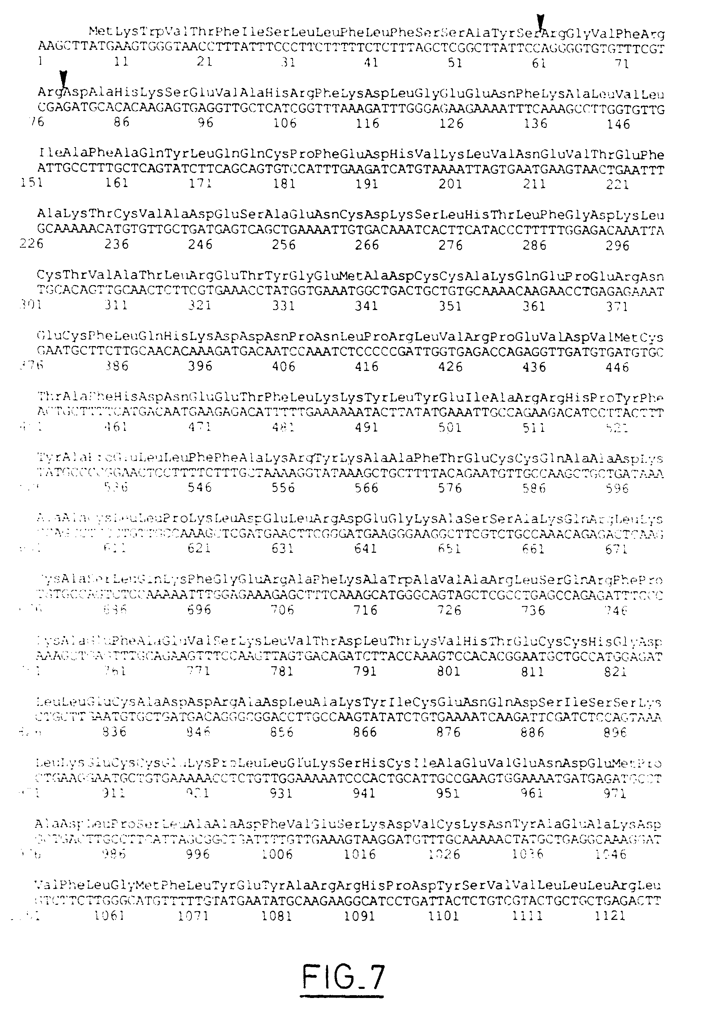

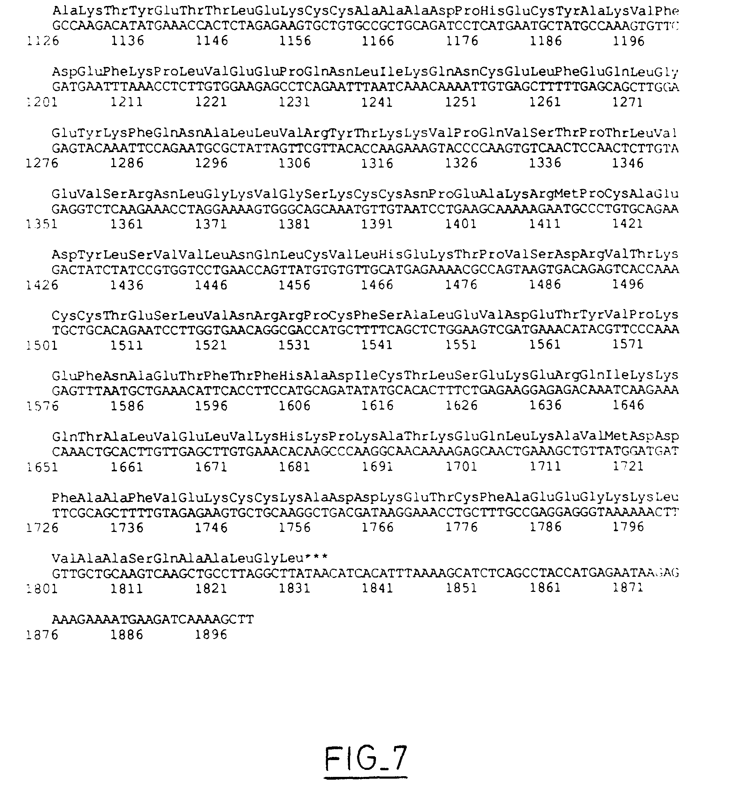

In order to obtain a prepro-HSA cassette capable of being readily integrated in expression vectors, the NdeI site of plasmid pXL322, described above, is changed into a HindIII site by oligodeoxynucleotide-directed mutagenesis. To this end, the HindIII-BglII fragment of pXL322 containing the 5' end of the prepro-HSA gene is subcloned into M13mp18 and mutagenized by hybridizing the synthetic oligodeoxynucleotide 5'-ATCTAAGGAAATAC AAGCTT**ATG**AAGTGGGT-3' to the single-stranded template (the underlined and bold-type sequences represent the HindIII site and the translation initiation site, respectively). Plasmid pXL855 was thus obtained (Figure 5), of which the nucleotide sequence of the mutagenized region was verified by using the dideoxy sequencing method. The sequence encoding the complete prepro-HSA is reconstituted by inserting the HindIII-PvuII fragment of the mutagenized phage and the PvuII-HindIII fragment of pXL322, containing the 3' end of the HSA structural gene, into the HindIII site of pUC8 to give plasmid pXL869 (Figure 6). This plasmid hence contains a 1.87 kb HindIII fragment containing the complete prepro-HSA structural gene as well as an untranslated 61 bp region at its 3' end. The complete sequence of the HindIII fragment as well as the amino acid sequence of the recombinant HSA, are depicted in Figure 7. A HindIII cassette containing the Met-HSA structural gene without any secretion signal is constructed by following the same procedure except that an M13 derivative of plasmid pXL276 is mutagenized using the synthetic oligodeoxynucleotide 5'-ATCTAAGGAAATACAAGCTT**ATG**GATGCACACAAG-3'. Reconstitution of the complete Met-HSA coding sequence in pUC8 gives plasmid pXL868, obtained in a similar manner to plasmid pXL869.

### Example 2: CONSTRUCTION OF YEAST CLONING VECTORS

### E.2.1 Isolation and purification of plasmid pKD1

Plasmid pKD1 may be purified from a late logarithmic phase culture of K. drosophilarum strain UCD 51-130 (U.C.D. collection, University of California, Davies, CA 95616) according to the following procedure which is derived from that described by Fleer et al. (Mol. Cell. Biol. 7 (1987) 1180 - 1192). A 1 liter culture in YPD medium (1% yeast extract, 2% Bacto-peptone (Difco), 2% glucose) is centrifuged, washed and resuspended in a 1.2 M sorbitol solution, and the cells are converted to spheroplasts in the presence of zymolyase (300 µg/ml), 25 mM EDTA, 50 mM phosphate and β-mercaptoethanol (1 µg/ml). After washing in 1.2 M sorbitol solution, the spheroplasts (corresponding to 250 ml of the original culture per tube) are resuspended in 2.5 ml of 1.2 M sorbitol and the same volume of buffer comprising 25 mM Tris-HCl, 50 mM glucose and 10 mM EDTA, pH 8.0, is added. The subsequent steps correspond to the alkaline lysis procedure already described (Maniatis, T. et al., "Molecular Cloning: a Laboratory Manual", Cold Spring Harbor Laboratory, Cold Spring Harbor, N.Y., 1982), except for the precipitation of the DNA which is carried out at 23°C for 15 minutes by adding 14 ml of isopropanol. The material obtained is treated with RNase (50 µg/ml) at 37°C for 20 minutes and then with proteinase K (150 µg/ml) in a solution comprising 0.5 M NaCl, 0.5% sarkosyl and 25 mM EDTA for 1 hour at 60°C. After centrifugation in an Eppendorf microcentrifuge, the supernatants are precipitated with ethanol for 10 minutes at -70°C, the pellets are then dissolved and the DNA is purified by CsCl density-gradient centrifugation in the presence of ethidium bromide.

### E.2.2 Construction of plasmid pCXJ1

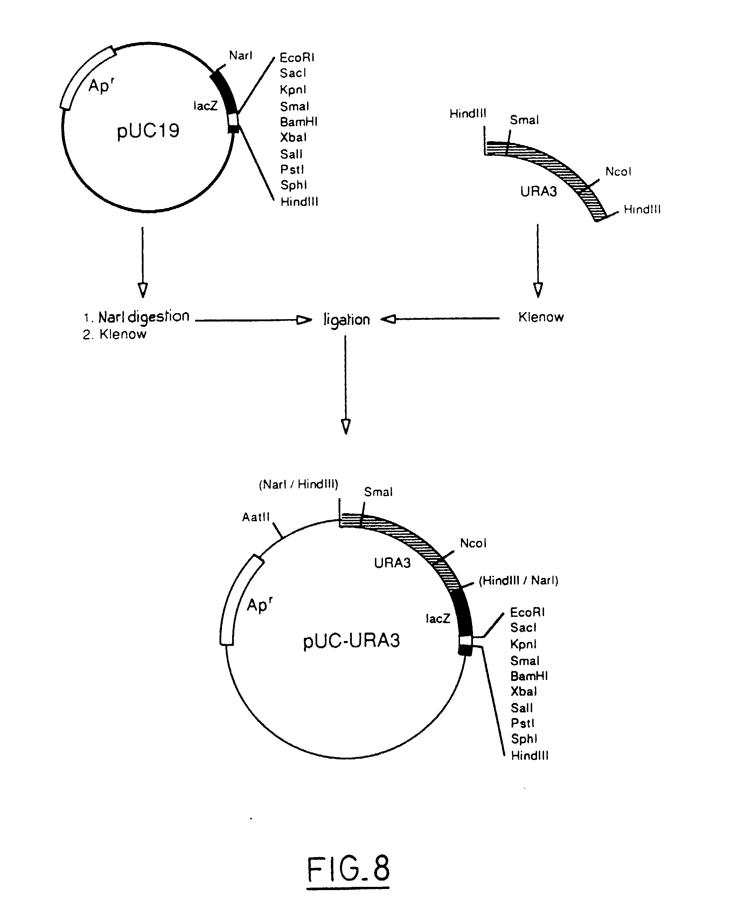

The construction intermediate pUC-URA3 (Figure 8) consists of a 1.1 kb HindIII fragment containing the URA3 gene of S. cerevisiae inserted into the unique NarI site of plasmid pUC19 (Yanisch-Perron, C. et al., Gene 33 (1985) 103 - 119). The HindIII fragment is derived from plasmid pG63 (Gerbaud, C. et al., Curr. Genet. 3 (1981) 173 - 180) which has been digested with HindIII and then treated with the Klenow fragment of E.coli DNA polymerase I to generate blunt-ended termini. The 1.1 kb fragment containing the URA3 gene is purified and then inserted into the plasmid pUC19, itself cut with NarI and treated with the Klenow fragment of E. coli DNA polymerase I. Plasmid pUC-URA3 hence contains: an origin of replication for maintenance of the plasmid in E. coli, the ampicillin resistance marker for transformations in E. coli, the lacZ gene containing a polylinker (EcoRI, SacI, KpnI, BamHI, XbaI, SalI, SphI, HindIII as unique sites) and the URA3 gene of S. cerevisiae serving as a selectable marker in uraA mutants of K. lactis.

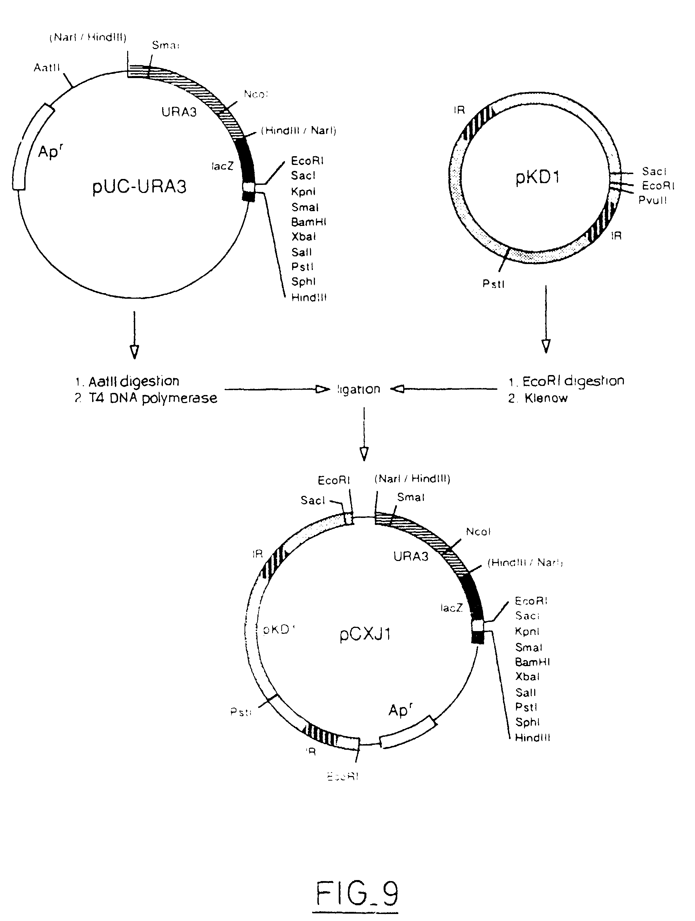

Plasmid pCXJ1 (Figure 9) contains the complete sequence of plasmid pKD1 inserted into the unique AatII site of pUC-URA3. This construction has been obtained by linearizing pKD1 at the EcoRI site and then treating this plasmid with the Klenow fragment of E. coli DNA polymerase I. This DNA fragment is ligated with pUC-URA3, previously cut with AatII and treated with T4 DNA polymerase. Ligation of these two fragments permits reconstitution of the EcoRI restriction sites. The insertion of the DNA of pUC-URA3 at the EcoRI site of the plasmid pKD1 does not inactivate any gene required for maintaining plasmid stability and copy number (the EcoRI site being located 205 nucleotides (nt) upstream from the ATG codon of gene B; Chen, X.J. et al., Nucl. Acids Res. 14 (1986) 4471 - 4481.). In consequence, plasmid pCXJ1, which transforms K. lactis uraA cir^{o} cells at high frequency, is amplified to about 70-100 copies per cell and stably maintained even in the absence of selection pressure. Due to the origin of replication contributed by pUC-URA3, plasmid pCXJ1 can also replicate in E. coli, thereby facilitating the plasmid construction and purification steps. The unique sites of plasmid pCXJ1 which can be used to insert foreign DNA and the HindIII and SalI sites from the pUC19 polylinker.

### E.2.3 Construction of a fusion between the K. lactis Pₖ₁ promoter and the 3'-aminoglycoside phosphotransferase gene of Tn903

The use of pCXJ1 as a vector for the transformation of K. lactis and other Kluyveromyces species remains limited to strains carrying the chromosomal uraA mutation as an auxotrophic marker. In order to be able to transform industrial wild-type strains of Kluyveromyces, the 3'-aminoglycoside phosphotransferase (aph) gene of the bacterial transposon Tn903 was chosen as a dominant antibiotic resistance marker which was inserted into pCXJ1. The aph gene confers resistance to kanamycin in E. coli, and if expressed in wild-type yeast strains confers resistance to the antibiotic G418 (geneticin), which is a potent inhibitor of cell growth (Jimenez, A. and Davis, J., Nature 287 (1980) 869-871). To permit a sufficiently strong expression of the aph gene in K. lactis, the bacterial transcription signals of the aph gene are replaced by the ORF1 promoter (Pₖ₁) isolated from the killer plasmid k1 of K. lactis.

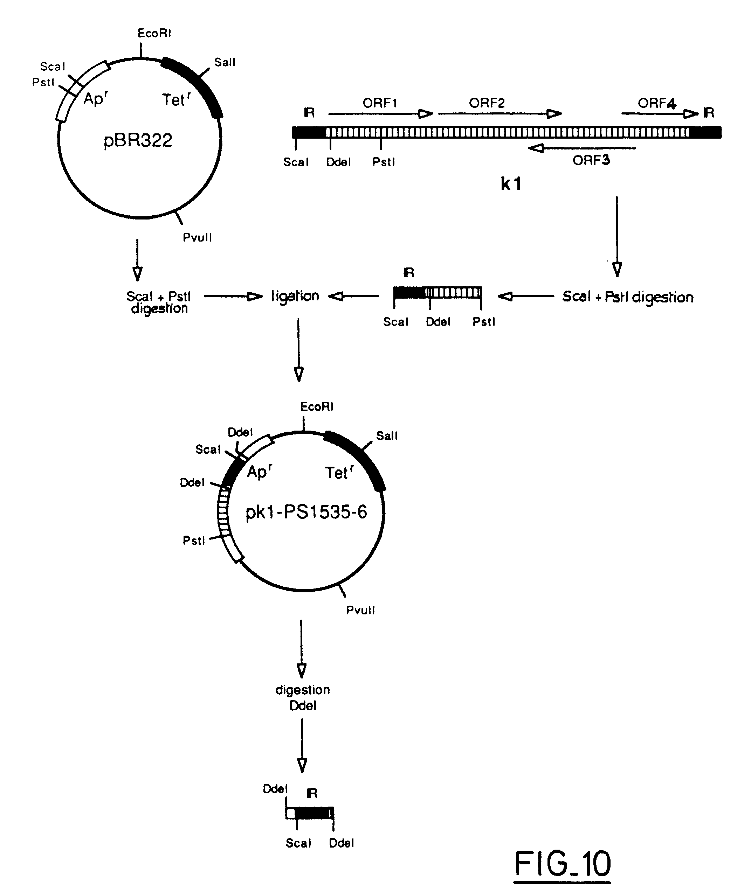

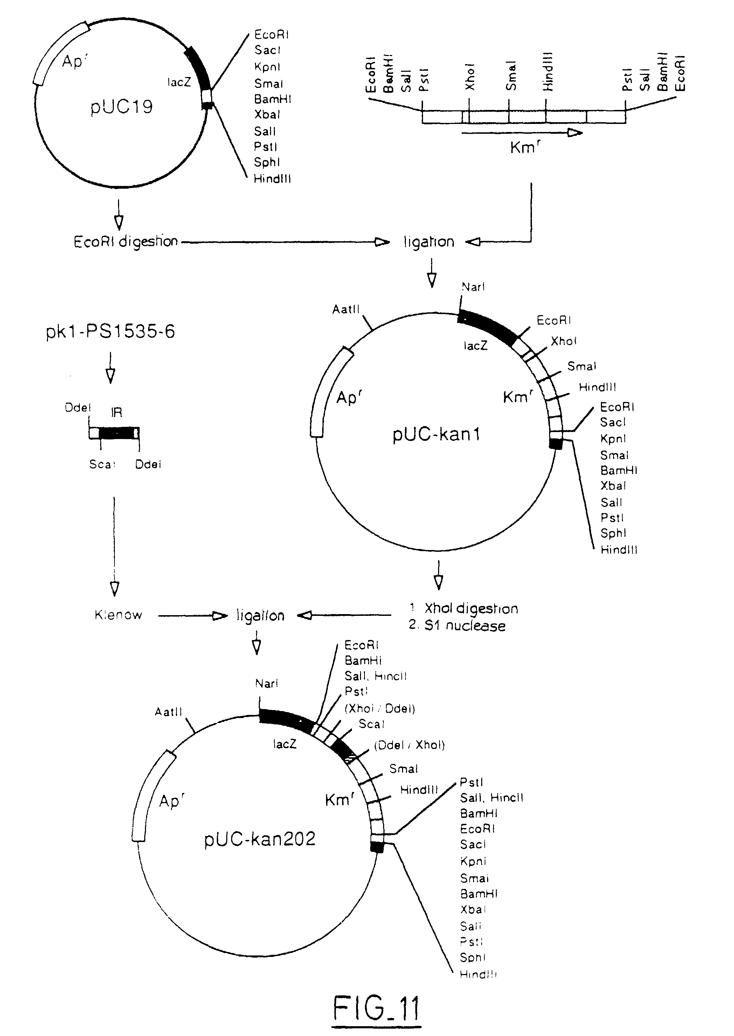

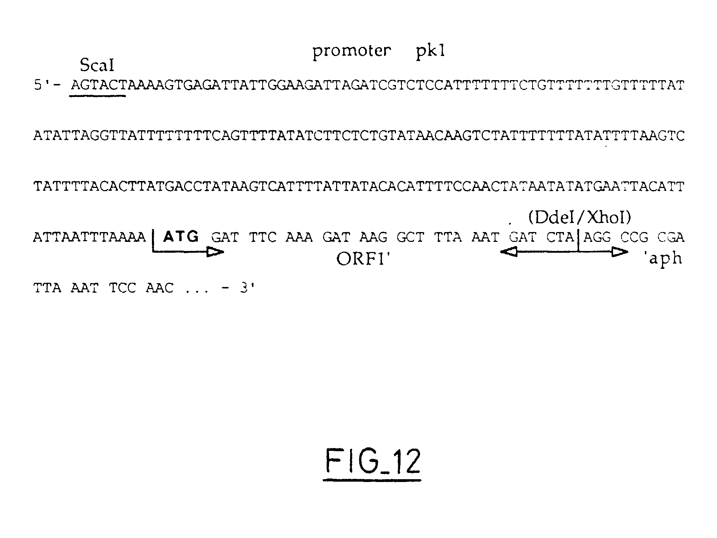

The construction of the Pₖ₁-aphfusion is performed in several steps (Figures 10 to 12). In the first place, a 1.5 kb ScaI-PstI fragment of plasmid k1 is subcloned between the unique ScaI and PstI sites of pBR322; the ampicillin-sensitive and tetracycline-resistant recombinant plasmid is designated pk1-PS1535-6 (Figure 10). The 1.5 kb ScaI-PstI subcloned fragment is derived from one extremity of the linear killer plasmid: it contains the 5' half of ORF1 carried by plasmid k1 and about 220 base pairs upstream (Sor, F. and Fukuhara, H., Curr. Genet. 9 (1985) 147 - 155). Since the ScaI site is located only 22 base pairs from the left-hand extremity of k1 (Figure 10), the 1.5 kb ScaI-PstI fragment probably contains the entire promoter region of ORF1 (Pk1). Digestion of pk1-PS1535-6 with DdeI yields a 266 bp fragment containing 17 bp derived from pBR322 at one of its extremities (close to ScaI) and the first 11 codons of ORF1 at the other extremity. After treatment with the Klenow fragment of E. coli DNA polymerase I, the purified fragment is inserted into the unique XhoI site of plasmid pUC-kan1 (Figure 11). The latter plasmid was obtained by inserting a 1.25 kb EcoRI fragment containing the aphgene derived from Tn903 (Kanamycin Resistance Gene Block™, Pharmacia) into the unique EcoRI site of pUC19. Plasmid pUC-kan202 is obtained by performing a digestion of pUC-kan1 with XhoI, followed by a brief digestion with S1 nuclease, rendering the termini blunt-ended, followed by ligation with the DdeI fragment of pk1-PS1535-6 rendered blunt-ended with the Klenow fragment of E. coli DNA polymerase I (Figure 11). This construction enables an in-frame fusion to be obtained between the first 11 amino acids of the ORF1 gene of the linear plasmid k1 and the 5' truncated end of the aph gene of Tn903. In fact, the junction between ORF1 and aph restores the twelfth codon of aph (AGG) so that the first eleven amino acids of aph have been replaced by the first eleven amino acids of ORF1. The complete sequence of the ORF1 promoter used for the Pₖ₁-'aph fusion together with the beginning of the structural gene encoding aph is shown in Figure 12.

### E.2.4. Construction of plasmid pKan707

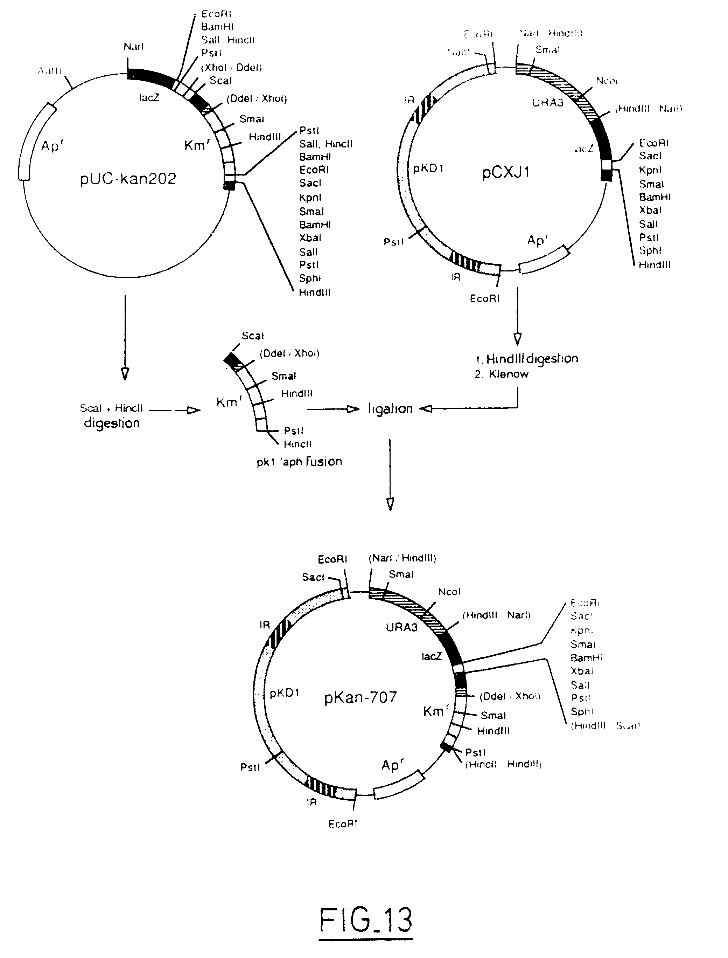

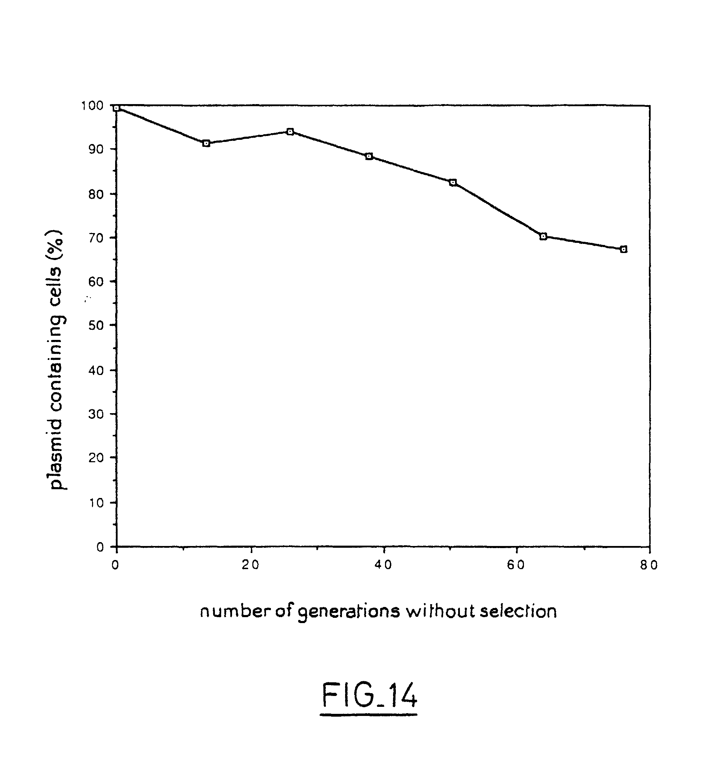

Plasmid pKan707 (Figure 13) is a derivative of plasmid pCXJ1 described above. It is constructed by cutting plasmid pCXJ1 with HindIII and then treating with Klenow fragment of E. coli DNA polymerase I. The linearized plasmid with blunt-ended termini is then ligated with a 1.2 kb ScaI-HincII fragment derived from plasmid pUC-Kan202 carrying the Pₖ₁-'aph fusion (Figure 13). Digestion of pUC-Kan202 with ScaI and HincII yields a blunt-ended kanamycin resistance cassette no longer containing the original bacterial promoter. The plasmid obtained, pKan707, confers resistance to very high levels of G418 (> 2.5 g/l) in K. lactis strains. As in the case of pCXJ1, plasmid pKan707 can be introduced into K. lactis cir^{o} strains at high frequency, amplified to 70 - 100 copies per cell and stably maintained in the absence of selection pressure (Figure 14). The great sensitivity to G418 of most Kluyveromyces strains combined with the presence of an efficient dominant marker permitting transformation of industrial strains makes pKan707 a very useful cloning vector for yeasts of the genus Kluyveromyces.

### Example 3: CONSTRUCTION OF EXPRESSION VECTORS CONTAINING THE EXPRESSION AND/OR SECRETION CASSETTES FOR ALBUMIN IN YEAST

### E.3.1 Construction of expression cassettes for Met-HSA and prepro-HSA under the control of the PGK promoter of S. cerevisiae.

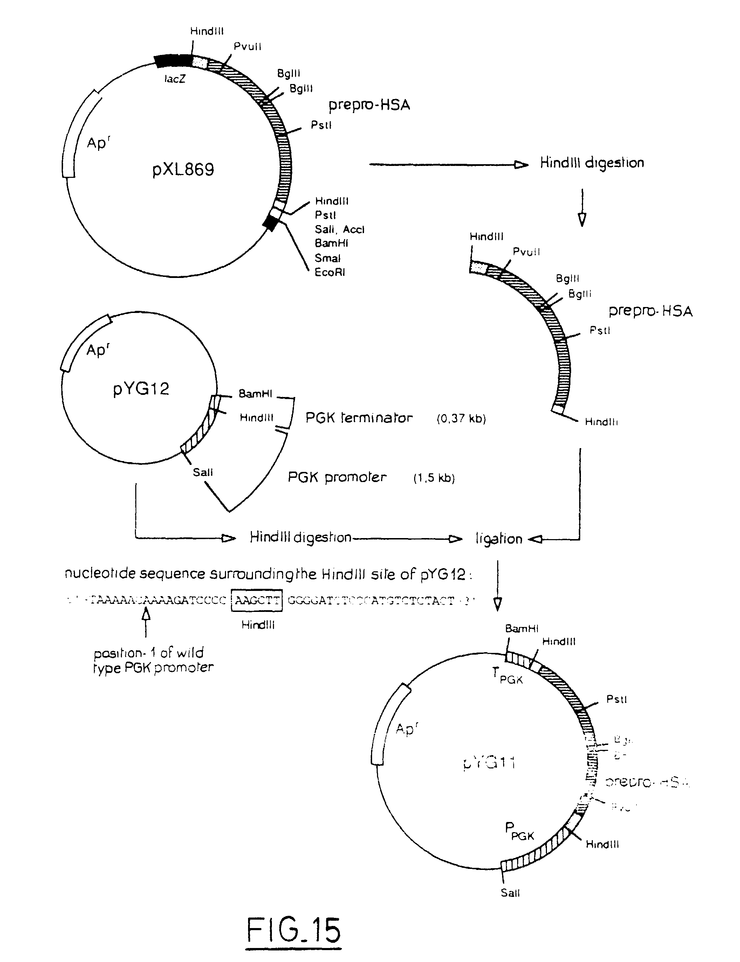

Plasmid pYG12 (Figure 15) contains a 1.8 kb SalI-BamHI restriction fragment consisting of the promoter and terminator regions of the PGK gene of S. cerevisiae. This fragment is derived from a 3.0 kb HindIII genomic fragment from which a 1.2 kb fragment corresponding to the structural gene has been deleted and comprising a region between the translation initiation ATG and the BglII site localized 30 codons upstream from the TAA codon specifying the termination of translation (Mellor, J. et al., Gene 24 (1983) 1 - 14). The HindIII sites flanking the 1.8 kb fragment which are thereby obtained are then destroyed and replaced by a SalI and BamHI site, respectively upstream from the promoter region and downstream from the PGK transcription terminator. A HindIII site is then introduced at the junction between promoter and terminator regions; since the site is unique, it enables heterologous genes to be readily introduced. The nucleotide sequence of this region is shown in Figure 15. The 1.8 kb HindIII fragment derived from plasmid pXL868 (see E.1.3) and encoding Met-HSA is then introduced into the HindIII site of plasmid pYG12 to give the recombinant plasmid pYG10. In a similar manner, plasmid pYG11 is generated by insertion into plasmid pYG12 of a HindIII fragment derived from plasmid pXL869 (see E.1.3) and encoding prepro-HSA (Figure 15). In consequence, two SalI-BamHI expression cassettes about 3.6 and 3.7 kb in size are thus constructed, comprising the PGK gene promoter of S. cerevisiae (P_{PGK}), followed by the gene encoding either Met-HSA or prepro-HSA, and finally the region corresponding to the transcription terminator of the PGK gene (T_{PGK}), a region including the polyadenylation site of the mRNA.

### E.3.2 Construction of expression plasmids pYG19, pYG23 and pYG221.

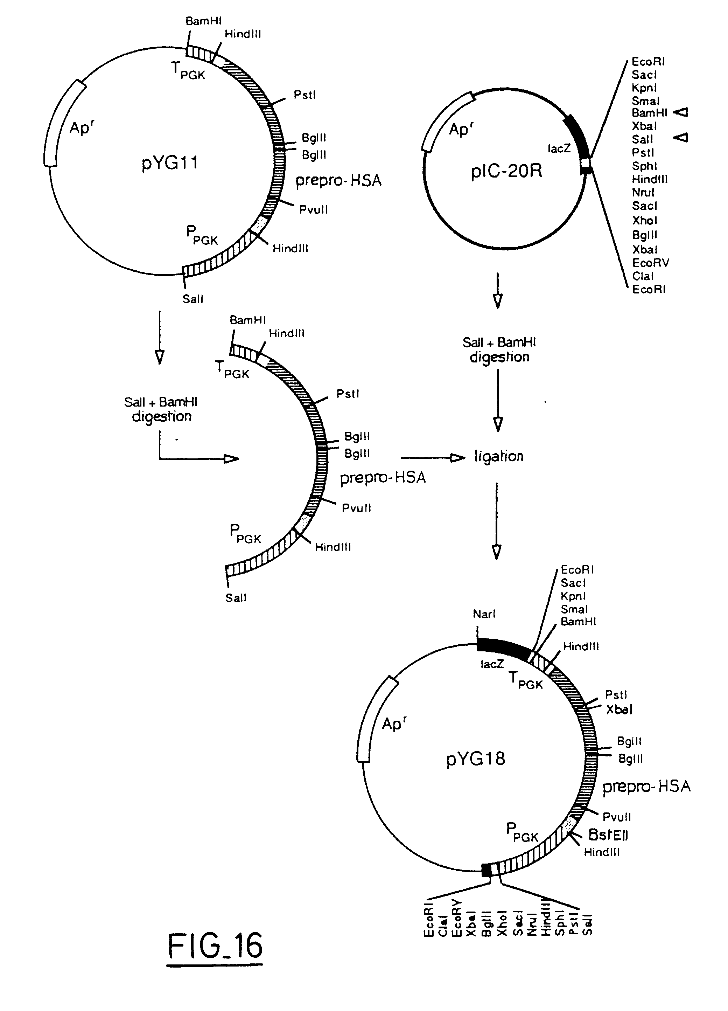

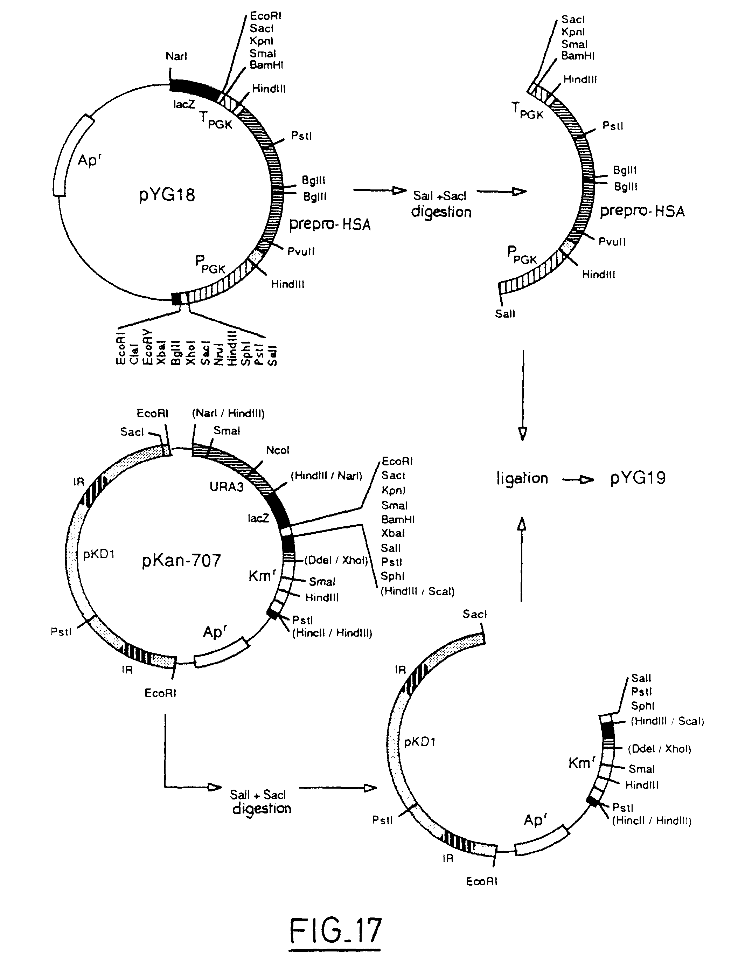

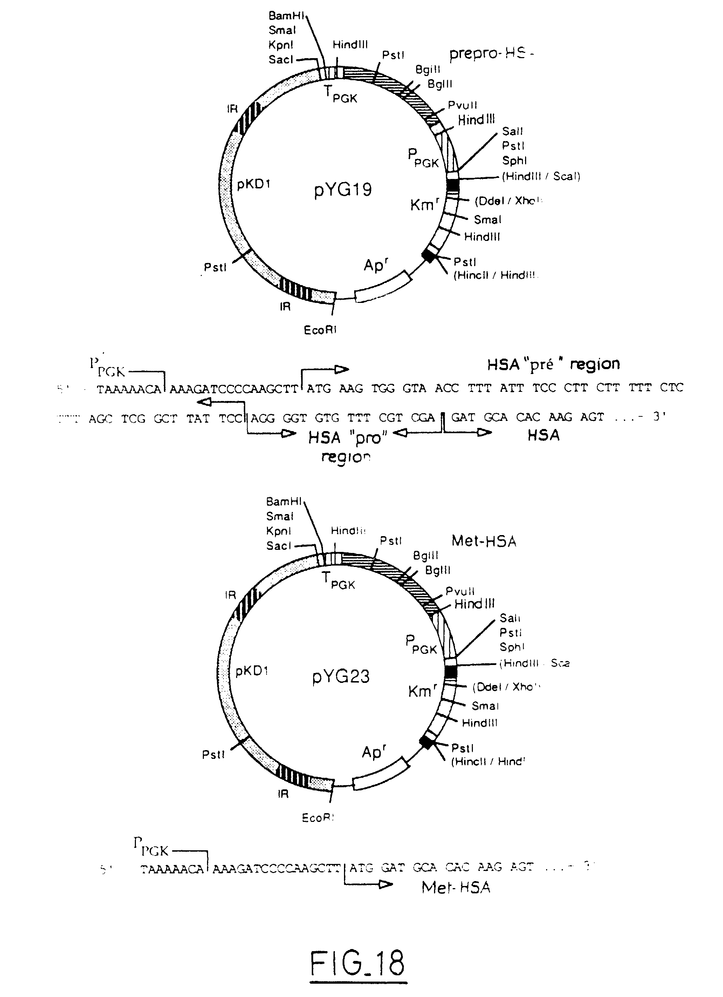

With the object of introducing the expression cassettes described above into the vector pKan707, the 3.6 and 3.7 kb SalI-BamHI fragments derived from plasmids pYG10 and pYG11 are, in a first stage, subcloned into the corresponding sites of plasmid pIC-20R (Marsh, L. et al., Gene 32 (1984) 481 - 485), to give in this manner the plasmid constructions pYG18 (prepro-HSA) (Figure 16) and pYG22 (Met-HSA). These construction intermediates give rise to P_{PGK}/Met-HSA/T_{PGK} and P_{PGK}/prepro-HSA/T_{PGK} expression cassettes in the form of SalI-SacI restriction fragments which can be directly inserted into the corresponding sites of pKan707 (Figure 17). Digestion of the latter with the enzymes SalI and SacI leads to deletion of the URA3 marker as well as that of a 35 bp fragment located upstream from gene B of pKD1. The constructions thereby obtained, designated pYG19 (prepro-HSA) (Figure 18) and pYG23 (Met-HSA) comprise the P_{PGK}/HSA/T_{PGK} expression cassettes, the virtually complete sequence of K. drosophilarum plasmid pKD1, the sequences permitting the autonomous replication in E. coli as well as the genes encoding β-lactamase and 3'-aminoglycoside phosphotransferase (under the control of the Pₖ₁ promoter), enabling, respectively, E. coli to be selected in the presence of ampicillin and K. lactis in the presence of G418.

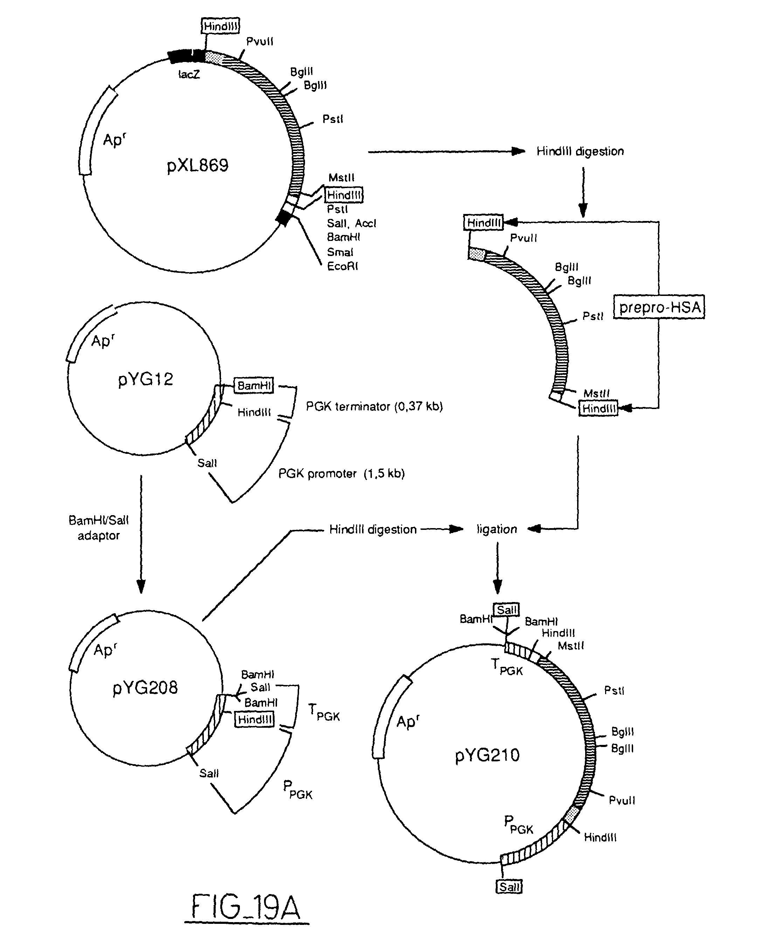

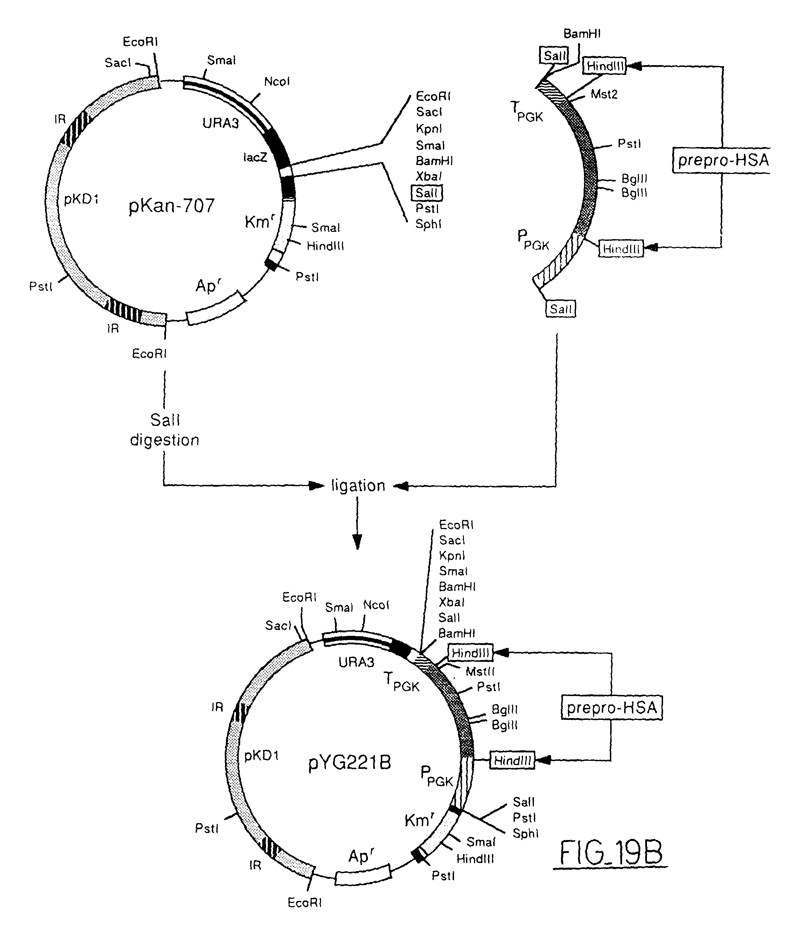

HSA-secretion vectors retaining the URA3 gene of plasmid pKan707 have also been constructed. As shown in Figure 19A, plasmid pYG208 is derived from plasmid pYG12 by inserting a BamHI/SalI adaptor (5'-GATCCGTCGACG-3') downstream from the PGK terminator. The HindIII fragment encoding prepro-HSA is purified by electroelution from plasmid pXL869 (prepro-HSA), and cloned in the correct orientation into the HindIII site of vector pYG208 to generate plasmid pYG210; the SalI expression cassette thereby obtained (PGK promoter/prepro-HSA/PGK terminator) is purified by electroelution and cloned into the SalI site of plasmid pKan707 to generate expression plasmids pYG221A and pYG221B (Figure 19B), which only differ from one another in the relative orientation of the SalI expression cassette with respect to the vector pKan707. In plasmid pYG221B, the orientation of the SalI expression cassette relative to the Km^{r} gene coding for resistance to G418 is identical to that of the SalI-SacI expression cassette carried by plasmid pYG19.

### E.3.3 Construction of a PGK/LAC hybrid promoter with optimized ATG context

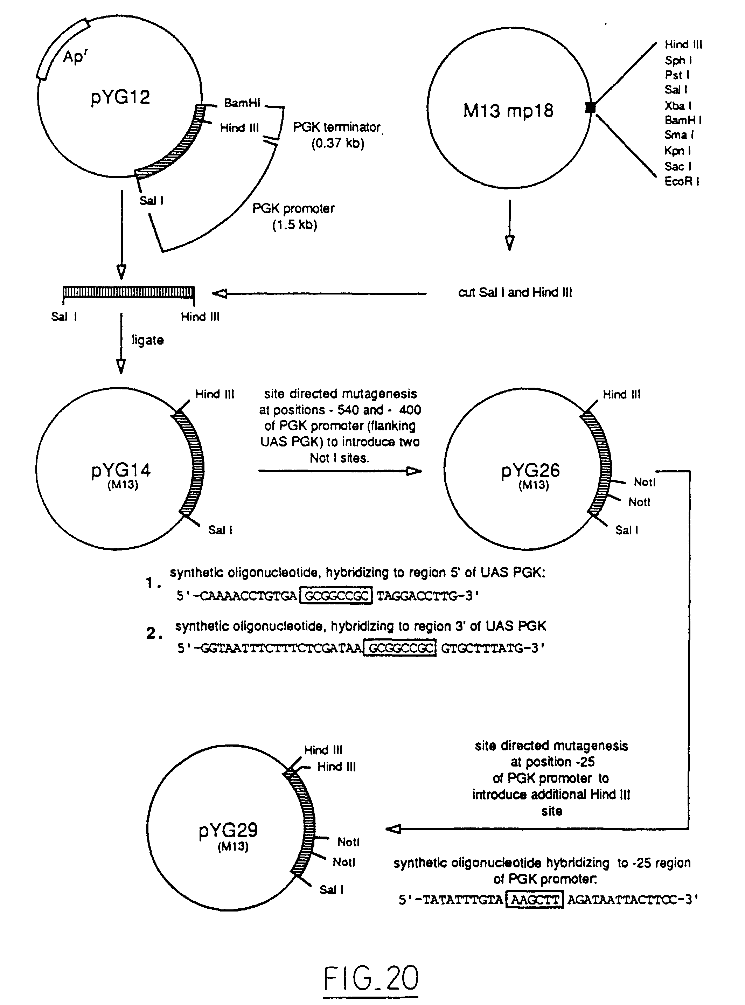

In order to obtain an inducible derivative of the PGK promoter present in expression plasmid pYG19, the UAS of the PGK promoter was replaced by the UAS derived from the LAC4 promoter of K. lactis (Breunig, K.D. et al., Nucl. Acid Res. 12 (1984) 2327 - 2341). To this end the SalI-HindIII fragment of pYG12 containing the PGK promoter was cloned into bacteriophage M13mp18 giving rise to construction pYG14 (Figure 20). Using the protocol described above, two NotI sites were then introduced into the PGK promoter region via site directed mutagenesis, generating construction pYG26 (Figure 20). The synthetic oligonucleotides used for this purpose were: 5'- CAAAACCTGTGAGCGGCCGCTAGGACCTTG-3' (Sq417) . and

5'- GG TAATTTCTTTCTCGATAAGCGGCCGCGTGCTTT ATG -3' (Sq416).

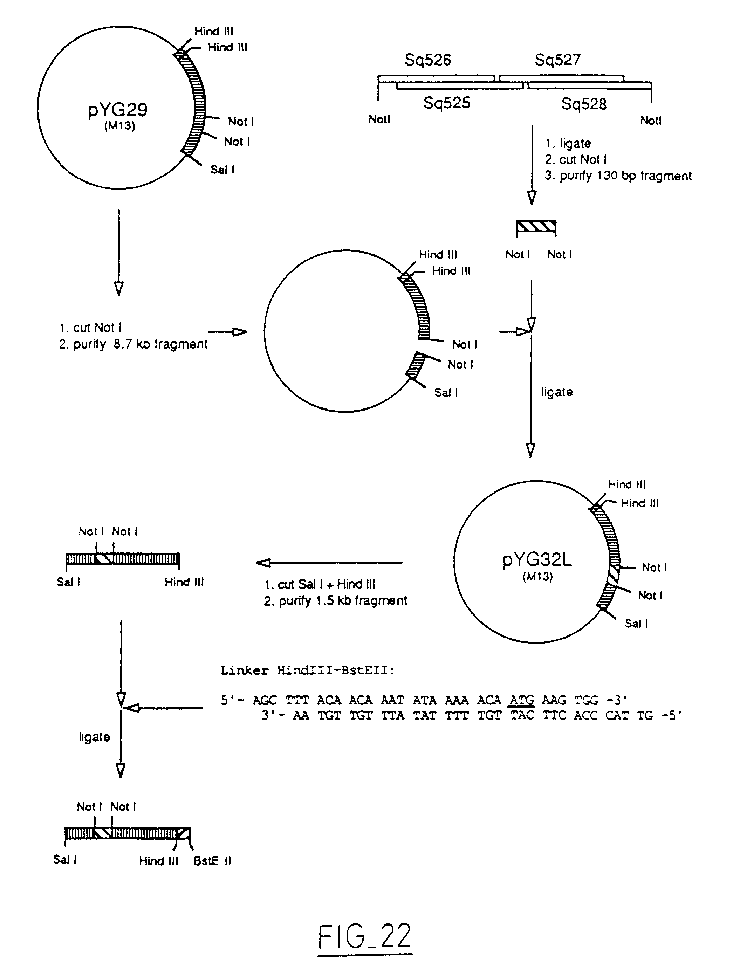

Thereby a total of four basepair changes (underlined) were introduced at positions -397, -399, -536, and -541 (with respect to the ATG initiator codon defined as +1; Figure 21). The two NotI sites (bold letters) obtained in this way flank the region of the PGK promoter which has previously been described to contain two functionally distinct domains (A and M in Figure 21 A) of the UAS_{PGK} (Stanway, C. et al., Nucl. Acids Res. 15 (1987) 6855 - 6873). Digestion of this PGK derivative with the enzyme NotI allows deletion of the UAS_{PGK} and its subsequent replacement by a synthetic fragment containing the UAS_{LAC} flanked by NotI sites (Figure 22), cloned in either orientation with regard to the PGK promoter. The UAS_{LAC} fragment was obtained by hybridization and subsequent ligation of the following four oligodeoxynudeotides:

Sq526, upper strand, fragment 1:
(-448) 5'-GGC CGC TGT GAA AAG TGT AGC GGA AAT ATG TGG TCC GAG
CAA CAG CGT CTT TTT CTA GTA GTG CGG-3' (-389)

Sq527, upper strand, fragment 2:
(-388) 5'-TCG GTT ACT TGG TTG ACA TTG GTA TTT GGA CTT TGT TGC
TAC ACC ATT CAC TAC TTG AAG TCG AGT GTG AGC-3' (-319)

Sq528, lower strand, fragment 1:

(-319) 5'-GGC CGC TCA CAC TCG ACT TCA AGT AGT GAA TGG TGT AGC
AAC AAA GTC CAA ATA CCA ATG TCA ACC AAG T-3' (-382)

Sq525, lower strand, fragment 2:
(-383) 5'-AAC CGA CCG CAC TAC TAG AAA AAG ACG CTG TTG CTC GGA
CCA CAT ATT TCC GCT ACA CTT TTC ACA GC-3' (-448)

Numbers in parentheses indicate the position of each oligodeoxynucleotide with respect to the ATG codon (+1) in the wild-type LAC4 promoter (Breunig, K.D. et al., Nucl. Acids Res., 12 (1984) 2327 -2341). The region spanning positions -328 to -435 has been shown to harbour the first of three UAS elements found in the LAC4 promoter (Breunig, K.D. et al., 5th Internat. Sympos. Genet. Industr. Microorgan. (1986) Alacevic M. et al. (eds.) 551 - 560; Leonardo, J.M. et al., Mol. Cell. Biol. 7 (1987) 4369 - 4376). Letters printed in bold face represent nucleotides which are part of the two NotI sites flanking the synthetic UAS of the LAC4 gene; the junction sequence of Sq527 complementary to Sq525 is underlined.

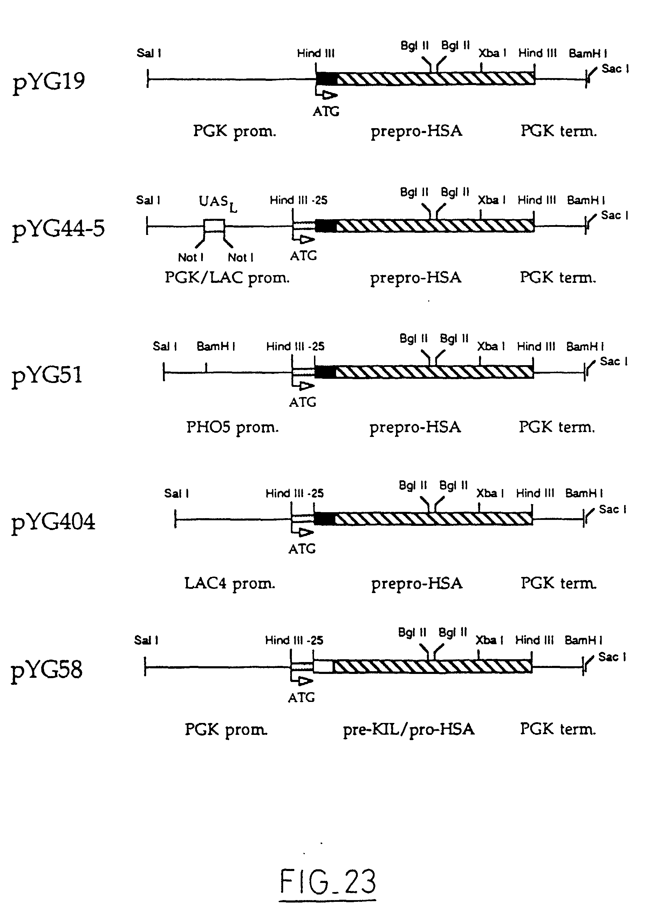

Since the nucleotide sequence near the initiator ATG of highly expressed eukaryotic genes is thought to influence the efficiency of translation initiation (Kozak, M., Microbiol. Rev. 47 (1983) 1 - 45; Hamilton, R. et al., Nucl. Acid Res. 15 (1987) 3581 - 3593), the ATG context of the PGK promoter was modified as follows: via site directed mutagenesis an additional HindIII restriction site (bold face) was introduced at position -25 of the PGK promoter carried on pYG26, thus generating plasmid pYG29 (Figure 20). The synthetic oligodeoxynucleotide used for this experiment was 5'-TATATTTGTTGTA **AAGCTT**AGATAATTACTTCC -3' (Sq449). The UAS_{LAC} fragment described above was then cloned into the NotI sites of pYG29 resulting in plasmids pYG63-1 and pYG63-2 (Figure 22). The PGK/LAC4 hybrid promoter regions of the latter plasmids were purified as SalI-HindIII fragments and ligated to a synthetic HindIII/BstEII adaptor composed of the following two complementary oligodeoxynucleotides: (i) 5'-AGC TTT ACA ACA AAT ATA AAA ACA ATG AAG TGG-3' (Sq451) and (ii) 5'-GT TAC CCA CTT CAT TGT TTT TAT ATT TGT TGT AA-3' (Sq452) (the prepro-HSA initiator codon is represented in bold letters). This adaptor reconstitutes the 22 bp immediately upstream of the wild-type PGK promoter (Figure 21 B) and comprises the first four codons of the prepro-HSA structural gene up to a naturally occuring BstEII site. The SalI-BstEII fragments obtained in this manner (carrying the PGK/LAC4 hybrid promoter with an optimized initiator ATG context) were then used to replace the corresponding restriction fragment (harbouring the non-mutagenized PGK promoter) in construction pYG18 (c.f. Figure 16). Following this strategy two construction intermediates were obtained which only differ in the orientation of the UAS_{LAC} with regard to the PGK promoter. These new constructs were used to generate SalI-SacI expression cassettes which were cloned into the corresponding restriction sites of pKan707 (c.f. Figure 13), generating plasmids pYG44-5 and pYG44-7 (Figure 23). These plasmids are isogenic except that the synthetic NotI fragment which harbours the UAS_{LAC} is in the wild-type orientation for pYG44-5 and in the opposite orientation for plasmid pYG44-7.

### E.3.4 Construction of a vector for prepro-HSA secretion under control of the PHO5 promoter of S. cerevisiae

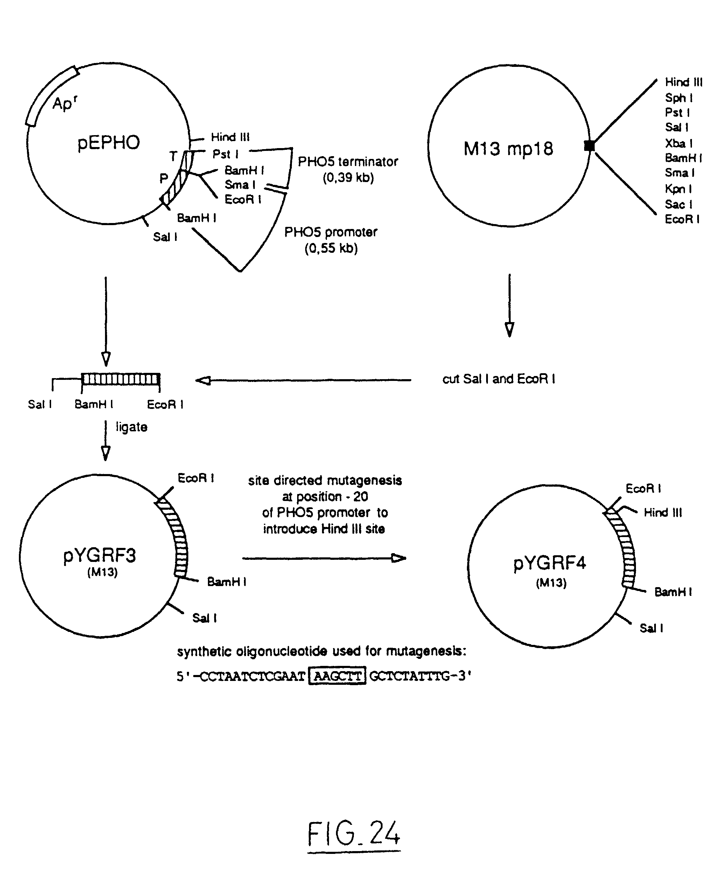

Vector pEPHO (Figure 24) contains a 0.94 kb BamHI-PstI restriction fragment harbouring the promoter and terminator region of the acid phosphatase gene (PHO5) of S. cerevisiae. This fragment is derived from a 2.03 kb genomic BamHI-PstI fragment (Bajwa, W. et al., Nucl. Acid Res. 12 (1984) 7721 - 7739) by deletion of the PHO5 structural gene. This deletion comprises positions -3 (with respect to the ATG initiator codon +1) and +1109 (the Sau3A site located upstream of the TAG terminator codon). A polylinker EcoRI/SmaI/BamHI was inserted at the junction between promoter and terminator and can be used as cloning site for the introduction of a heterologous gene. The junction between the PHO5 promoter and this polylinker is given below (the nucleotide sequence corresponding to the polylinker is underlined):

5'-AAATTCGAGATTAGGAATTCCCGGGGATCC-3'

In order to obtain a construct in which the HSA gene is expressed under control of the PHO5 promoter and the non-translated leader region of the PGK gene, a HindIII site was introduced at position -20 of the PHO5 promoter fragment derived from plasmid pEPHO. To this end, the 0.83 kb SalI-EcoRI fragment of pEPHO was subcloned into the polylinker of bacteriophage M13mp9 and the resulting single-stranded template (pYGRF3) was then mutagenized using the oligodeoxynucleotide 5'-CCTAATCTCGAAT**AAGCTT** GCTCTATTTG-3' (Sq487; the HindIII site introduced is represented in bold characters) as mutagenic primer, resulting in plasmid pYGRF4 (Figure 24).

To facilitate the construction of a variety of expression cassettes containing different promoters and/or terminators, a derivative of plasmid pIC-20R (c.f. Figure 16) was generated in which the HindIII site of the polylinker was destroyed by treatment of the cohesive ends with the large fragment of polymerase I (Klenow) of E. coli. This new cloning vector (pIC-20RΔH3) was then used to introduce the SalI-BamHI fragment of plasmid pYG12 (c.f. Figure 15) containing the promoter and terminator regions of the PGK gene, thus generating plasmid pYG61. By using promoter modules as SalI-HindIII fragments and terminator modules as HindIII-BamHI fragments, expression cassettes can be generated with any combination of transcriptional initiation and termination signals into which the structural gene to be expressed has been cloned as a HindIII restriction fragment. Digestion of these construction intermediates with the enzymes SalI and SacI allows subsequent introduction of the new expression cassettes into the corresponding sites of yeast cloning vector pKan707 (c.f. Figures 17 and 18).

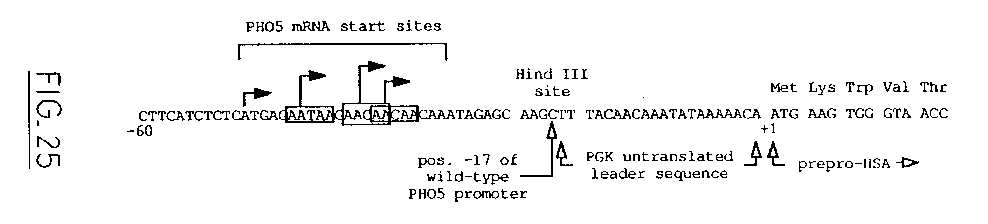

Following this basic strategy, the SalI-HindIII PGK promoter fragment of plasmid pYG61 was replaced by the SalI-HindIII fragment of pYGRF4 harbouring the PHO5 promoter. Insertion of the prepro-HSA structural gene isolated from plasmid pYG44-5 as HindIII fragment (c.f. Figure 23) and transfer of the resulting expression cassette as SalI-SacI fragment into plasmid pKan707 gave rise to secretion vector pYG51 (Figure 23). This plasmid thus contains the PHO5 promoter of S. cerevisiae up to position -17 (including all transcription initiation sites (Rudolph, H. and Hinnen, A., Proc. Natl. Acad. Sci. USA 84 (1987) 1340 - 1344), Figure 25), followed by 21 bp of the untranslated PGK leader sequence, the prepro-HSA structural gene, and finally the PGK terminator.

### E.3.5 Construction of a vector for HSA secretion under control of the LAC4 promoter of K. lactis

Using the polymerase-catalyzed chain reaction (PCR) technique, the LAC4 promoter of K. lactis was amplified from yeast genomic DNA as a SalI-HindIII restriction fragment. Total genomic DNA isolated from strain CBS2359 served as template and the following synthetic oligodeoxynucleotides were used as primers for the Taq-polymerase reaction (the SalI and HindIII sites introduced upstream and downstream, respectively, of the LAC4 promoter of K. lactis are represented in bold letters):

1) 5'-CCCGTCGACATGTAGAGTAGACAACAGACAGGGAGGGC -3'

2) 5'-AAAGCTTATCTTTCAGTTCTCGATGAGTATGTGTGTT -3'

The design of these primers was based on the LAC4 sequence previously published by Leonardo J.M. et al. (Mol. Cell. Biol. 7 (1987) 4369 - 4376). The SalI-HindIII restriction fragment obtained in this manner contains the LAC4 promoter region spanning positions -1198 to -1 (the ATG initiator codon being defined as +1). The amplified material was digested with the enzymes SalI and HindIII, cloned into plasmid pYG61 (c.f. section E.3.4), and its nucleotide sequence was confirmed using the dideoxy chain termination method.

Following the general scheme described in the previous section a pKan707 derivative was obtained, designated pYG404 (Figure 23), in which the prepro-HSA gene is expressed under the control of the lactose/galactose inducible LAC4 promoter of K. lactis. The sequence immediately upstream of the HSA initiator codon in this specific construction is derived from plasmid pYG44-5 (fragment HindIII) and therefore contains part of the untranslated leader of the PGK gene (c.f. section E.3.3).

### E.3.6 Construction of a vector for HSA secretion under control of the PGK promoter of S. cerevisiae and the killer toxin secretion signal of K. lactis

Using two pairs of complementary synthetic oligodeoxynucleotides we reconstituted a HindIII-DraI fragment which contains the following elements: (i) the ATG proximal 21 bp of the PGK promoter, (ii) the secretion signal sequence ("pre"-region) of the killer toxin gene from plasmid k1 of K. lactis (Stark M.J.R. and Boyd A., EMBO J. 5 (1986) 1995- 2002), and (iii) the first 17 amino acids of the pro-HSA gene up to the N-terminal DraI site, fused in frame to the killer toxin pre-region. The four oligodeoxynucleotides used for this construction were as follows:

Sq1000, upper strand, fragment 1:
5'-AGCTTAGCTTTACAACAAATATAAAAACAATGAATATATTTTACATA
TTTTTGTTTTTGCTGTC-3'

Sq998, upper strand, fragment 2:

5'-ATTCGTTCAAGGTAGGGGTGTGTTTCGTCGAGATGCACACAAGAGTGA
GGTTGCTCATCGGTTT-3'

Sq996, lower strand, fragment 1:
5'-AAAAACAAAAATATGTAAAATATATTCATTGTTTTATATTTGTTGT
AAAGCTA-3'

Sq997, lower strand, fragment 2:
5'-AAACCGATGAGCAACCTCACTCTTGTGTGCATCTCGACGAAACACACC
CCTACCTTGAACGAATGACAGC-3'

Letters printed in bold face represent nucleotides which are part of the HindIII and DraI sites used in this experiment; the junction sequence of Sq998 complementary to Sq996 is underlined.

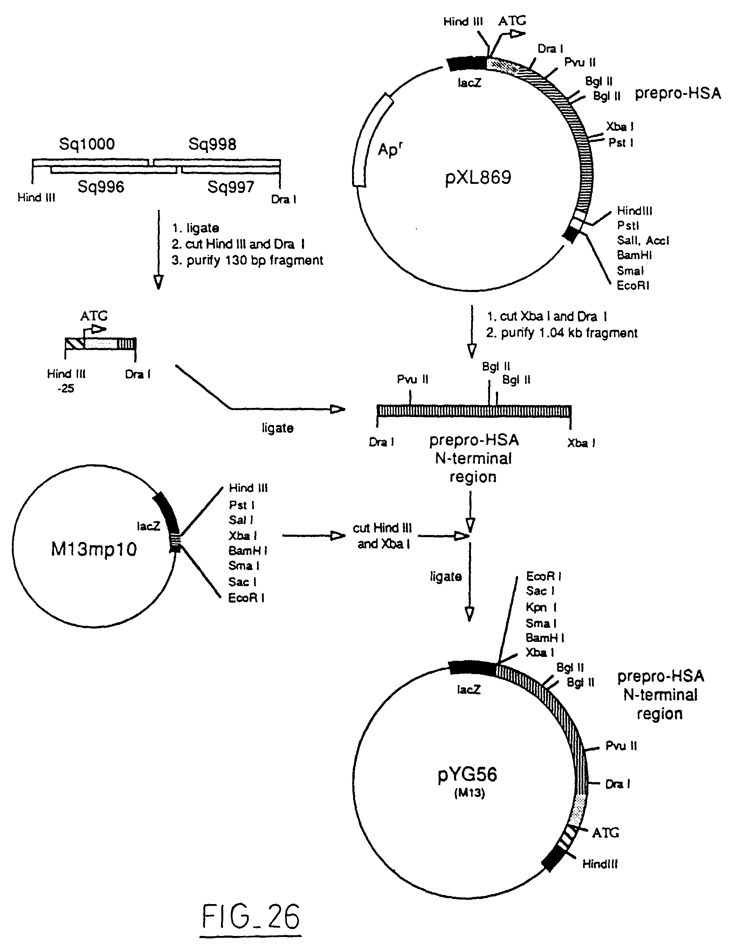

After phosphorylation of oligodeoxynucleotides Sq996 and Sq998, a double stranded HindIII-DraI fragment was generated according to the scheme depicted in Figure 26. This 130 bp fragment was purified by gel electrophoresis and ligated to a 1.04 kb DraI-XbaI fragment containing part of the HSA structural gene isolated from plasmid pXL869 (c.f. Figure 6). The HindIII-XbaI fragment obtained in this manner was then cloned into bacteriophage M13mpl0, generating construction pYG56 (Figure 26). The correct nucleotide sequence of this intermediate was confirmed by dideoxy sequencing.

The entire prepro-HSA structural gene can then be reconstituted by ligation of the following three elements: (i) the 1.17 kb HindIII-XbaI fragment derived from the pYG56 construct just described, (ii) the XbaI-BamHI fragment of pYG18 (c.f. Figure 16) containing the carboxy-terminal half of the HSA gene as well as the PGK terminator region, and (iii) a BamHI-HindIII fragment of plasmid pYG62. This latter plasmid is a derivative of construction pYG61 (c.f. section E.3.4) in which the SalI-HindIII fragment carrying the PGK promoter has been replaced by the equivalent restriction fragment derived from plasmid pYG29 (c.f. Figure 20). The BamHI-HindIII fragment of plasmid pYG62 used in this construction thus contained the E. coli origin of replication, the ampicillin resistance gene, and the PGK promoter with a HindIII site introduced at position -25 (c.f. section E.3.4). The resulting pIC derivative was designated pYG57. This latter plasmid was used as source of a SalI-SacI fragment which was cloned into the corresponding sites of yeast cloning vector pKan707 giving rise to expression vector pYG58 (Figure 23).

### E.3.7 Construction of an integrative vector for directing a PGK/prepro-HSA secretion cassette to the RAG2 locus of K. lactis

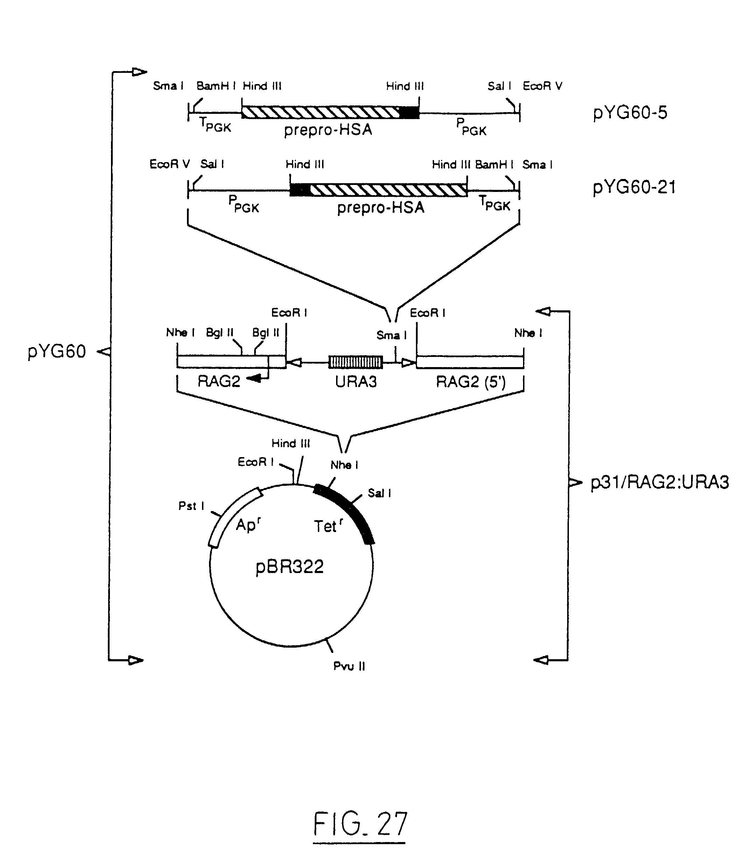

Plasmid p31/RAG2:URA3 (Figure 27), kindly provided by Dr. M. Wésolowski-Louvel (Institut Curie, Orsay, France), contains a 2.3 kb genomic NheI fragment harbouring the promoter and N-terminal region of the RAG2 structural gene of K. lactis which most likely encodes a phosphoglucose isomerase (Wésolowski-Louvel M. et al., Nucl. Acid Res., 16 (1988) 8714). Plasmid p31/RAG2:URA3 was constructed by insertion of a 1.6 kb EcoRI fragment derived from plasmid pCXJ1 (c.f. Figure 9), harbouring the URA3 gene of S. cerevisiae, into the EcoRI site of the RAG2 promoter. The 3.9 kb NehI fragment obtained in this way was then cloned into the unique NheI site of plasmid pBR322.

By cutting plasmid pYG18 (Figure 16) with the restriction enzymes EcoRV and SmaI a 3.7 kb fragment was obtained containing the same PGK/prepro-HSA expression cassette as the one used in yeast secretion vector pYG19 (Figure 18). Insertion of this cassette into the unique SmaI site of plasmid p31/RAG2:URA3 (located 3' of the URA3 marker gene) generated constructions pYG60-21 and pYG60-5 (Figure 27) which only differ from each other with respect to the orientation of the prepro-HSA expression cassette.

By cutting plasmids pYG60-21 and pYG60-5 with the enzyme NheI a 7.7 kb fragment was generated containing the HSA expression cassette and the URA3 marker gene flanked by RAG2 derived sequences. These latter sequences served to target the integration of the entire fragment to the RAG2 locus of K. lactis strain MW98-8C via homologous recombination (Rothstein R.J., Methods in Enzymol. 101 (1983) 202 - 211). Integration at the RAG2 locus was confirmed by Southern analysis for three out of six integrants tested (MW98-8C::60-5 integrant clone #6 and MW98-8C::60-21 integrants clones #7 and 9). The remaining three integrants were found to carry one (MW98-8C::60-5 integrant clone #8 and MW98-8C::60-21 integrant clone #11) or two (MW98-8C::60-5 integrant clone #4) HSA expression cassettes integrated at loci other than RAG2. All six of the confirmed integrants were tested for efficiency of HSA secretion.

### Example 4: TRANSFORMATION OF KLUYVEROMYCES SPECIES WITH PLASMIDS EXPRESSING HSA

### E.4.1 Transformation procedures

Transformants of K. lactis strain MW98-8C (α uraA arg lys K⁺ pKD1°) were obtained either by the spheroplast formation technique initially described by Hinnen et al. (Hinnen, A. et al., Proc. Natl. Acad. Sci. USA 75 (1978) 1929 - 1933) and appropriately adapted, or by treating the whole cells with lithium acetate (Ito, H. et al., J. Bacteriol. 153 (1983) 163 - 168), which favors DNA incorporation in the presence of polyethylene glycol (PEG 4000, Sigma). Transformants of all other Kluyveromyces strains described below were exclusively obtained by treatment with lithium acetate.

The modifications relating to spheroplast formation have already been described (Bianchi, M. et al., Curr. Genet. 12 (1987) 185 - 192). When the method involving lithium acetate is used, cell growth is performed at 28°C in 50 ml of YPD medium with agitation, and to an optical density at 600 nm (OD₆₀₀) between 0.6 and 0.8. The cells are harvested by low-speed centrifugation, washed in sterile TE solution (10 mM Tris-HCl pH 7.4, 1 mM EDTA), resuspended in 3-4 ml of lithium acetate solution (0.1 M in TE) to give a cell density of about 2 x 10⁸ c/ml and then incubated at 30°C for 1 hour with moderate agitation.

0.1 ml aliquot portions of the resulting suspension of competent cells are incubated at 30°C for 1 hour in the presence of DNA and PEG 4000 at a final concentration of 35%. After a thermal shock of 5 minutes at 42°C, the cells are washed twice with sterile water, resuspended in 0.2 ml of sterile water and transferred to 10 ml tubes. YPD medium containing 0.7% agar and kept molten at 46°C is then added (5ml) and the mixture is immediately poured onto YPD plates. After solidification, an additional overlayer of 5 ml of top agar is then added. After a 18 to 24 hours incubation at 28°C, 0.16 ml of G418 solution (Geneticin 50 mg/ml, GIBCO, Grand Island, N.Y.) is spread onto the plates and transformants are counted after 4-5 days of additional incubation at 28°C.

Direct plating of the cells on YPD + G418 plates (i.e. omitting the use of top agar) results in the appearance of clones of larger size for the transformed K. lactis cells. However, a lower concentration of G418 (final concentration 50 µg/ml) has to be used to observe colonies, which leads to the selection of mutants partially resistant to G418 and derived from non-transformed cells. In addition, the transformation efficiency is at least ten times lower than the efficiency observed after phenotypic expression of the resistance gene for 18 to 24 hours.

### E.4.2 Mitotic stability of HSA-expressing pKD1 derived plasmids under non selective growth conditions

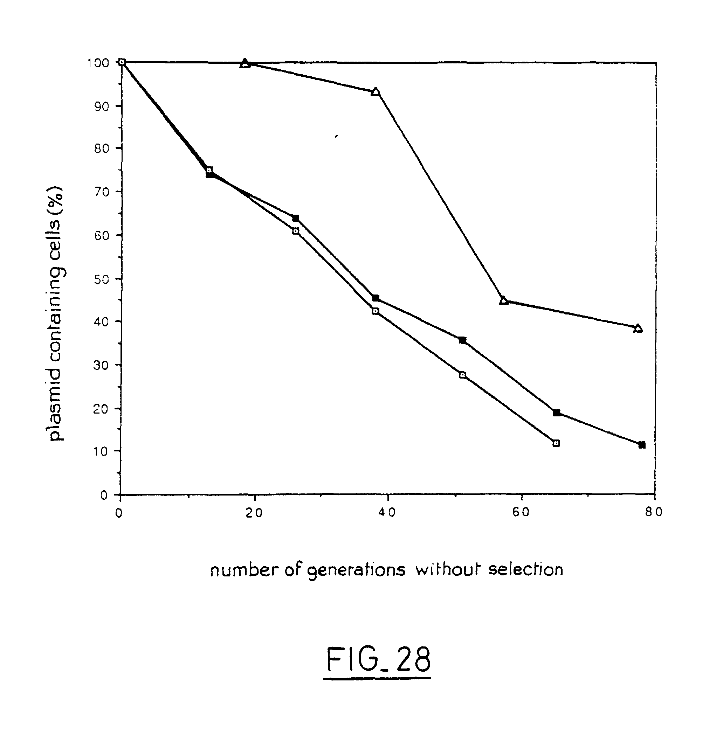

The mitotic stability of plasmids pYG19 and pYG23 is measured at different time intervals after growth in nonselective medium; it is determined as the ratio between the final and the initial percentages of cells growing on YPD plates containing 200 µg/ml of G418. As shown in Figure 28, both plasmids are exceptionally stable despite the high level of expression of a heterologous gene. When transformants of strain MW98-8C are grown in a non-selective medium for 40 cell generations, 40-45% of the cells have maintained these plasmids. Plasmid pYG19 shows even higher mitotic stability in K. lactis strain CBS683: over 80 % of the cells maintain the plasmid after 40 generations of non-selective growth.

### E.4.3 pYG19 and pYG23 transformants of K. lactis MW98-8C: HSA expression and secretion

The expression and secretion levels of K. lactis MW98-8C cells containing plasmids pYG19 (prepro-HSA) and pYG23 (Met-HSA) are determined after different time intervals after growth at 28°C in non selective YPD medium and with constant agitation. The culture supernatants are obtained by two consecutive centrifugations (5 minutes at 4000 rpm in a Kontron Hermle Z-365 K centrifuge) and then 10 minutes at 12000 rpm) in order to eliminate all possible contamination by cells. A sample of the second supernatant (0.5 ml) is heated to 95°C for 15 minutes in presence of an equal volume of sample buffer containing 0.125 M Tris-HCl, 20% glycerol, 10% 2-mercaptoethanol (β-ME), 4.6% sodium dodecyl sulfate (SDS) and 0.4% bromophenol blue. When a concentration of the proteins present in the supernatant is desired, 0.4 ml of a 100% w/v trichloroacetic acid solution (TCA) is added to 8 ml of supernatant and the proteins are precipitated on ice for 1 hour. The precipitated material is recovered by centrifugation for 20 minutes at 15000 rpm and then resolubilized by heating to 95°C for 15 minutes in 0.5 ml of sample buffer containing 63 mM Tris-HCl, 10% glycerol, 5% β-ME, 2.3% SDS and 0.2% bromophenol blue.

The intracellular expression of albumin is detected using cell extracts prepared as follows: the equivalent of 0.25 ml of a cell pellet (washed once in saline buffer) is resuspended in the same volume of lysis buffer kept on ice and containing 67 mM phosphate, pH 7,5 mM β-ME, 1 mM phenylmethylsulfonyl fluoride (PMSF), 2 µM leupeptin and 2 µM pepstatin A (Sigma). After addition of 0.5 ml of zirconium beads (0.5 mm diameter) stored in phosphate buffer (67 mM, pH 7) at 4°C, the cells are ruptured by 7 consecutive 30 second periods with a cell disrupter (Biospec Mini Bead-Beater, Biospec Products) interspersed by 2 minute cooling periods on ice. In these conditions, the efficiency of cell disruption is more than 90% as judged by counting the cells under a phase-contrast microscope. The liquid fraction bereft of zirconium beads is transferred to an Eppendorf tube and the beads are washed 3 times with 0.2 ml of lysis buffer and then collected in the same Eppendorf tube. By centrifugation of the tube for 15 minutes at 4°C and at 12000 g, a soluble protein fraction (supernatant) and an insoluble protein fraction (pellet) are thus defined. These two fractions are taken up to give the same final volume of sample buffer. These samples are then heated for 15 minutes to 95°C and aliquotes are layered on an 8.5% SDS-polyacrylamide gel preceded by a 5% stacking gel (Laemli, U.K., Nature 227 (1970) 680 - 685), and subjected to a current of 25 mA until the bromophenol blue reaches the bottom of the gel.

Figure 29 shows the result of a typical experiment which enables an assessment to be made of the expression and secretion of albumin obtained with K. lactis strain MW98-8C transformed with plasmids pYG19 (prepro-HSA), pYG23 (Met-HSA) and pYG25 (vector devoid of an expression cassette), after growth in 50 ml of YPD medium without G418 at 28°C for 68 hours. Each sample corresponds to 100 µl of original culture and enables the soluble fractions, insoluble fractions and culture supernatants to be compared after migration in 8.5% polyacrylamide gel and Coomassie Blue stain. A protein band which comigrates with commercial human albumin (Sigma) serving as a molecular weight reference, is detectable in the samples derived from the cells transformed with plasmids pYG19 or pYG23, but not with cells containing the vector pYG25 which does not contain the HSA gene. It will be noted that, whereas virtually the whole of the albumin expressed using pYG19 (prepro-HSA) is exported into the culture supernatant, all the albumin produced using plasmid pYG23 (Met-HSA) is present in the insoluble cytoplasmic protein fraction. In addition, the results in Figure 29 show that albumin excreted by the cells containing plasmid pYG19 is the only extracellular protein present in significant amounts. In the supernatant of a shake flask culture of MW98-8C transformed with plasmid pYG19 and grown in appropriate conditions (c.f. section E.4.6.), the albumin concentration can reach 150 mg HSA/liter.

### E.4.4 Immunological detection of the albumin produced by K. lactis.

The identity of the yeast proteins which co-migrate with albumin standard may be tested by immunological detection. To this end, a polyacrylamide gel is blotted onto a nitrocellulose filter (Schleicher and Schuell, 0.45 µm) using a semi-dry blotting apparatus (Biometra) in a transfer buffer containing 25 mM Tris base, 150 mM glycine and 10% methanol. The blotting time is 30 minutes using a current of approximately 0.85 mA/cm² of gel surface. After blotting, the filter is incubated for three times 5 minutes with 50 ml of buffer A (5% skimmed milk powder, 0.2% Tween 20 in PBS buffer [137 mM NaCl, 2.7 mM KCl, 4.3 mM Na₂HPO₄]), followed by incubation for 30 minutes in 40 ml of buffer A containing rabbit polyclonal antibodies directed towards HSA and diluted 1:1000. After 3 rinses of the filter with buffer A, a second, biotinylated antibody recognizing the rabbit antibodies (Vectastain ABC Immuno Peroxidase Kit, Vector Laboratories), is added on the basis of one drop per 50 ml of buffer A. After incubation of the filter for 30 minutes, the filter is rinsed 3 times with buffer B (0.2% Tween 20 in PBS) and then incubated in the presence of an avidin DH/biotinylated peroxidase H complex. The avidin/biotinylated peroxidase complex is prepared immediately before use by diluting 1 drop of each of reagents A and B of the kit in 5 ml of buffer B after incubation at 23°C for 30 minutes. The filter is incubated in a 1:10 dilution of the complex in buffer B (50 ml in total) for 30 minutes. The filter is rinsed again with buffer B for 3 5-minute periods and developed for 2-3 minutes in a solution of 10 ml containing 0.02% H₂O₂ and 10 ml of a solution containing 1 mg/ml of diaminobenzidine and 0.4 mg/NiCl₂ in 0.1 M Tris-HCl pH 7.4. Figure 30 shows that K. lactis MW98-8C expresses (pYG23) and excretes (pYG19) a protein recognized by anti-HSA polyclonal antibodies. This antigenic material is specifically associated with the presence of an albumin-expressing cassette since it is not detected in cell extracts or culture supernatants from yeasts transformed with the vector without an expression cassette.

### E.4.5 Kinetics of HSA secretion

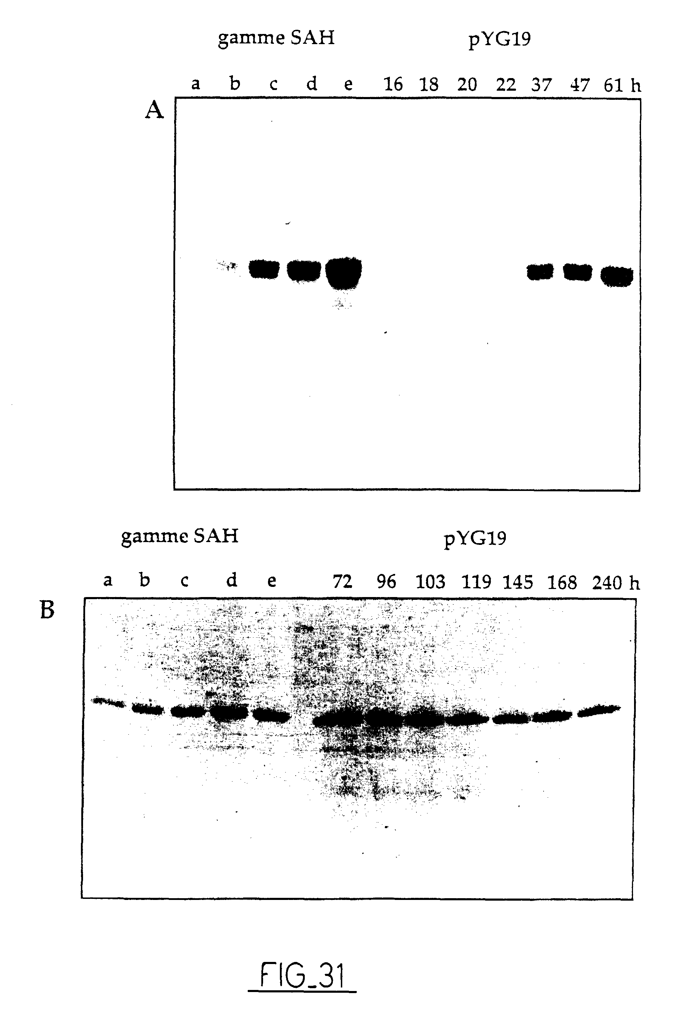

As shown in Figure 31A, the secretion of albumin takes place with relatively slow kinetics in an Erlenmeyer. The maximum excretion levels appear to take place after 70 to 100 hours of incubation of a culture originally inoculated at 10⁵ cells/ml (Figure 31B). No significant increase or decrease is detectable between 100 and 240 hours of culture, suggesting a good stability of HSA under these growth and temperature conditions. Since there is no accumulation of proteolytically degraded material during this period, it may be concluded that no extracellular protease which might degrade the excreted albumin is present.

A graphic representation of the kinetics of excretion (A), as well as the growth curve of the strain MW98-8C transformed with plasmid pYG19 (B) is shown in Figure 32; these results show that the excretion of albumin continues after the cells have reached the stationary growth phase. This observation is in agreement with observations relating to other expression systems in yeast (Tschopp, J.F. et al., Bio/Technology 5 (1987) 1305-1308).

### E.4.6 Efficiency of HSA secretion with plasmids pYG44, pYG51, pYG404, and pYG58.

The efficiency of HSA secretion has been tested under control of the PGK promoter of S. cerevisiae (plasmids pYG19; Figures 29 to 32), a PGK/LAC4 hybrid promoter with optimized ATG context (plasmids pYG44-5 and pYG44-7; Figure 33), the PHO5 promoter of S. cerevisiae (plasmid pYG51; Figure 34 A), and the LAC4 promoter of K. lactis (plasmid pYG404; Figure 35). In addition, the secretion signal sequence (pre-region) of the killer toxin of K. lactis has been fused in frame to the pro-HSA structural gene (plasmid pYG58; Figure 34 B) and the secretion efficiency of the fusion protein compared to that of the natural prepro-HSA (for details of plasmid constructions c.f. example 3). The results obtained with these constructions can be summarized as follows:

The level of HSA secreted into the culture medium when expressed from the PGK/LAC4 hybrid promoter present in plasmid pYG44 is increased two to three fold if cells are cultured in glucose containing media to which lactose is added between mid-log and early stationary growth phase (Figure 33). This effect is not observed if the addition of lactose is replaced by an equivalent amount of glucose (data not shown). If cells are cultured in media containing lactose as sole carbon source, levels of secreted HSA are not higher as the ones obtained with glucose containing media (Figure 33 B). The lactose mediated increase of HSA secretion observed after "induction" of cells subsequent to initial cultivation in glucose, however, is not dependent on the presence of the UAS element of the LAC4 promoter of K. lactis. This lactose mediated increase in HSA secretion is equally found when HSA expression is directed by the PGK promoter (Figure 33 A, B for strain MW98-8C transformants; Figure 35 for CBS2359 transformants) or the PHO5 promoter of S. cerevisiae (data not shown).

HSA secretion under control of the LAC4 promoter of K. lactis results in lower HSA levels as the ones obtained with the PGK promoter of S. cerevisiae (Figure 35). On the other hand, LAC4 directed HSA expression is tightly regulated: no secreted HSA could be detected in culture supernatants of CBS2359 transformants when glucose was the only available carbon source (Figure 35).

Surprisingly, when cells are grown in complete medium (YPD), the efficiency of PHO5 directed HSA secretion is only slightly below the one observed with constructs containing the glycolytic PGK promoter (Figure 34 A).

The replacement of the HSA secretion signal by the pre-region of the killer toxin of K. lactis does neither influence the kinetics nor the efficiency of HSA secretion (Figure 34).

### E.4.7 Efficiency of HSA secretion with integrated expression cassettes.

The PGK/prepro-HSA expression cassette present in plasmid pYG19 has been integrated into the K. lactis chromosomal DNA by the one step gene disruption method (for experimental details c.f. section E.3.7). As shown in figure 36, the levels of HSA secreted into the culture medium by the integrant strains (lanes 2-7) are at least twenty fold below those obtained by the pKD1 derivative pYG19 (lane 1). HSA secretion was basically the same whether integration of the expression cassette occured at the RAG2 locus or elsewhere in the K. lactis genome (Figure 36). Furthermore, the orientation of the expression cassette with regard to the URA3 selectable marker gene did not have a significant influence on HSA secretion levels (Figure 36, lanes 2 and 3). However, a twofold increase of secreted HSA was observed with transformants which contained two integrated expression cassettes per genome (Figure 36, lane 5). In summary, the efficiency of HSA secretion appears to be directly linked to the number of copies of the structural gene carried in a transformed yeast cell.

### E.4.8 Secretion of HSA in a variety of species and strains of the genus Kluyveromyces.

Plasmids pYG19, pYG221B or pKan707 have been used to transform the following strains of Kluyveromyces: ATCC16045 (K. marxianus var. bulgaricus), ATCC24178 (K. wickerhamii), ATCC12424 (K. marxianus var. marxianus), ATCC56500 (K. waltii), ATCC36906 (K. marxianus var. drosophilarum), CBS4574 (K. marxianus var. lactis), CBS683 (K. marxianus var. lactis), and MW98-8C (K. marxianus var. lactis). Detectable levels of secreted HSA were obtained with all transformants examined, even though appreciable variations were observed among different strains. The highest levels were found using transformants of strains MW98-8C (CBS579.88) and CBS683, the lowest level was observed with strain ATCC16045 where it could only be detected using immunological methods (data not shown). Plasmids pYG19 and pYG221B mediated comparable levels of secreted HSA in all strains tested.

### Example 5: PURIFICATION OF HSA SECRETED INTO THE GROWTH MEDIUM

In a typical purification experiment, strain MW98-8C transformed with plasmid pYG19 is grown in YPD medium for 72 hours under the standard conditions already described. A culture supernatant (0.5 liter) is freed from all cellular contamination by centrifugation, and incubated at 4°C for 15 minutes in the presence of 60% ethanol (V/V). The precipitate is recovered by centrifugation, redissolved in 10 ml of a solution comprising 50 mM Tris-HCL pH 8.0, and then loaded onto a Trisacryl blue column (I.B.F., France). The recombinant albumin is eluted from this column with 3M NaCl solution. After dialysis of the fractions containing HSA against 50 mM Tris-HCl, these fractions are purified on MONO Q column (Pharmacia), and eluted at a NaCl concentration of 0.25 M. In a final stage, chromatography on Superose 12 (Pharmacia) enables the HSA to be obtained at more than 99% purity as judged from the silver staining of SDS polyacrylamide gels.

### Example 6: CHARACTERIZATION OF THE SECRETED AND PURIFIED ALBUMIN

The lack of a test enabling the biological properties of albumin to be measured in vitro does not provide an easy mean to assess the quality of the recombinant albumin. For this reason, after purification, the albumin excreted by K. lactis is hence characterized by several physicochemical and immunological tests. These tests demonstrate that the recombinant albumin is secreted by K. lactis in a mature form and in its native configuration: it is not distinguishable from human serum albumin extracted from plasma with respect to any of the criteria applied.

### E.6.1 PAGE-SDS: Coomassie Blue and Silver staining.

Electrophoresis is performed on a SDS polyacrylamide gel (7.5%) (Figure 38 C) as described above, or by using the"Phast gel" system (Pharmacia, Figure 38 A). Different amounts of recombinant albumin were compared with a commercial preparation of standard albumin (Sigma) extracted from human plasma. Staining of the gel with Coomassie Blue (Figure 38 C) or with silver salts (Figure 38 A) shows the complete homogeneity of the albumin samples from yeast.

### E.6.2 Isoelectrofocusing.

Isoelectric focusing is performed between pH 4.5 and 6.0 (Phast gel, Pharmacia, Figure 38 B), and between pH 4.0 and 7.0 (Immobiline, LKB). The isoelectric point of the recombinant HSA is identical to that of the reference human HSA (pI = 4.8).

### E.6.3 Native PAGE and immunoblotting.

Electrophoresis of the recombinant HSA in a non-denaturing polyacrylamide gel (10%), followed by blotting onto a nitrocellulose filter and immunodetection under the conditions described in section E.4.4, reveal a comigration with the standard albumin extracted from human plasma. This suggests very strongly that correct maturation of the recombinant prepro-HSA takes place during the process of secretion in K. lactis . No contamination with non-maturated HSA is detectable, which implies that cleavage of the signal sequence and of the pro-sequence of HSA takes place efficiently in this yeast.

### E.6.4 Molecular sieving chromatography

Molecular sieving chromatography is performed with Superose 12 (Pharmacia). The flow rate of the elution buffer (50 mM Tris-HCl pH 8.0) is maintained at 1 ml/minute. The concentrations of recombinant albumin and of reference albumin are 0.4 and 1 mg/ml respectively. The elution of albumin is detected by measuring the absorbance at 280 nm. In both cases, the elution volume is identical (11.5 ml).

### E.6.5 Anion exchange chromatography

The recombinant albumin is eluted from a Mono Q HR 5/5 column (Pharmacia) at a concentration of 0.31 M NaCl, as is the reference albumin derived from human plasma. Figure 39 shows the chromatographic profiles obtained for injections of 100 µl of recombinant HSA (0.4 mg/ml) and of reference HSA (0.5 mg/ml) using a column equilibrated with 50 mM Tris-HCl pH 8.0, and at a constant flow rate of 1 ml/minute.

### E.6.6 Reversed-phase chromatography

The behavior of the yeast albumin in reversed-phase is analyzed on a Nucleosil (C4) column with buffers A (water containing 0.1% trifluoroacetic acid (TFA)) and B (acetonitrile containing 0.1% TFA). Figure 40 shows the elution, with a gradient of 20 to 80% of B in A, of the yeast albumin, which exhibits the same retention time as the reference albumin.

### E.6.7 Amino acid composition

The amino acid composition of the recombinant HSA is determined by reversed-phase chromatography after acid hydrolysis (6 N HCl) and phenylisothiocyanyl derivatization. The results obtained by this method show clearly an identical composition to that of the reference albumin derived from human plasma.

### E.6.8 N-Terminal sequence

The use of an automated apparatus for Edman degradation (Applied Biosystems) shows that the N-terminal sequence of the HSA secreted by K. lactis is Asp-Ala-His-Lys-Ser-Glu-Val-Ala-His-Arg-Phe-Lys-Asp-Leu-Gly. The determination of this sequence hence confirms the results derived from the native polyacrylamide gel electrophoresis experiments, and demonstrates the correct and complete maturation of the albumin secreted by K. lactis.

### E.6.9 Tryptophan fluorescence

After excitation at 295 nm, the fluorescence emission of the single tryptophan shows the same profile for both recombinant and plasma albumins (maximum at 337 nm), indicating an identical hydrophobic environment around this amino acid (Figure 41).

### E.6.10 Thermal stability

The reference albumin (Sigma) and the albumin secreted by the yeast have identical stability kinetics at 75° C (Figure 42), which suggests that the bonds stabilizing the protein structure (hydrophobic interactions and disulfide bonds) are identical in both cases.

### E.6.11 Reactivity towards monoclonal antibodies

The antigenicity of the recombinant albumin was studied with different monoclonal antibodies directed towards human albumin. Figure 43 shows their specificity: the antibodies HA10, HA11 and HA13 correspond to epitopes whose conservation requires the integrity of the whole molecule. The antibodies HA21, HA6, HA4, HA3, HA8 and HA1 correspond to epitopes localized along the peptide chain from the N-terminal to the C-terminal end (Doyen, N. et al., Mol. Immun. 22 (1985) 1 - 10; Lapresle, C., Anal. Biochem. 174 (1988) 308 - 312).

The test employed is an ELISA (Enzyme-Linked Immuno-Sorbent Assay) inhibition test. The different antibodies are adsorbed on polystyrene plates and a binding curve of albumin labeled with alkaline phosphatase is established for each antibody. The saturation curves for each antibody have a sigmoid appearance, and the amount of labeled albumin corresponding to 50% binding was chosen to study the inhibition by each antibody.

Inhibition was carried out with the sample of recombinant albumin and compared with that obtained with a sample of plasma albumin (Sigma). The pannels A, B, and C of Figure 44 shows that, for the nine antibodies tested, the recombinant albumin is as inhibitory as native albumin. Given the extreme sensitivity of the test, the differences observed are not likely to be significant.

### Note

A sample of strain MW98-8C has been deposited at the Centraalbureau voor Schimmelkulturen (CBS) at Baarn in the Netherlands according to the provisions of the Treaty of Budapest, where it was registered on 16.09.1988 under number CBS 579.88.

## Claims

1. A method for a preparation of human serum albumin (HSA), in which method a yeast of the genus Kluyveromyces transformed with an expression plasmid comprising:
- functional genes A, B and C of plasmid pKD1,
- the inverted repeats of plasmid pKD1,
- the stability locus of plasmid pKD1,
- the origin of replication of pKD1 and an expression cassette containing a no-yeast DNA encoding the structural gene for human serum albumin under the control of sequences permitting its expression in said yeast,
- a selectable marker for the transformed yeast,
- and, optionally, an origin of replication and a selectable marker for Escherichia coli,
is cultured in a growth medium.

2. The method as claimed in claim 1, wherein the expression cassette and the selectable marker are inserted in the EcoRI site of pKD1.

3. The method as claimed in one of claims 1 and 2, wherein the expression cassette and the selectable marker are inserted in a region of 197 nucleotides defined by the Saci and MstII sites of pKD1, or the SphI site of pKD1.

4. The method as claimed in one of claims 1 to 3, wherein the sequences permitting the expression of the structural gene are chosen from the promoters derived from genes of yeasts of the genus Saccharomyces or Kluyveromyces.

5. The method as claimed in claim 4, wherein these promoters are derived from the glycolytic genes of yeasts of the genus Saccharomyces or Kluyveromyces.

6. The method as claimed in claim 4, wherein the sequences permitting the expression of the structural gene are chosen from the genes encoding phosphoglycerate kinase (PGK), glyceraldehyde-3-phosphate-dehydrogenase (GPD), enolases (ENO), alcohol dehydrogenases (ADH), lactase (LAC4) or acid phosphatase (PHO5).

7. The method as claimed in claim 4, wherein the sequences permitting the expression of the structural gene are chosen from the genes encoding phosphoglycerate kinase (PGK) or acid phosphatase (PHO5).

8. The method as claimed in one of claims 1 to 7, wherein the sequences encoding human serum albumin are preceded by a protein-exportation sequence which is chosen from the natural N-terminal leader of human serum albumin.

9. The method as claimed in one of claims I to 8, wherein the sequence encoding human serum albumin is preceded by an exportation sequence different from the natural sequence.

10. The method as claimed in claim 9, wherein the sequence encoding the exportation signal of human serum albumin is the sequence obtained from yeast genes encoding the alpha pheromone or killer toxin.

11. The method as claimed in one of claims 1 to 10, wherein the sequence permitting the excretion of human serum albumin is the prepro natural terminal leader of albumin.

12. The method as claimed in one of claims 1 to 11, wherein the selectable markers for the transformed yeasts are chosen from the genes providing resistance to antibiotics or to copper ions.

13. The method as claimd in claim 12, wherein the selectable sequence is a gene providing resistance to G418.

14. The method as claimed in claim 13, wherein the selectable sequence is a fusion between the ORF1 promoter of the linear plasmid kl of K. lactis and the aminoglycoside phosphotransferase of the transposon Tn903.

15. The method as claimed in one of claims 1 to 14, wherein the selectable marker is a gene complementing auxotrophies.

16. The method as claimed in one of claims I to 15, wherein the strain is of the species K. marxianus, K. wickerhamii, K. waltii.

17. The method as claimed in claim 16, wherein the strain is of the species K. bulgaricus, K. marxianus var. drosophilarum K. marxianus var. marxianus or K. marxianus var. lactis.

18. The method according to anyone of claims 1 to 17, wherein yeasts are cultured in glucose containing medium to which lactose is added between mid-log and early stationary growth phase.

## Patentansprüche

1. Verfahren zur Herstellung von Humanserumalbumin (HSA), wobei in diesem Verfahren eine Hefe der Gattung Kluyyeromyces, die transformiert ist mit einem Expressionsplasmid, welches umfaßt:
- funktionelle Gene A, B and C des Plasmids pKD1,
- die invertierten Sequenzwiederholungen (inverted repeats) des Plasmids pKD1,
- den Stabilitätslocus des Plasmids pKD1,
- den Replikationsursprung von pKD1 und eine Expressionskassette, die Nicht-Hefe-DNA (no-yeast DNA), welche für das strukturelle Gen für Humanserumalbumin kodiert, unter der Kontrolle von Sequenzen enthält, die seine Expression in dieser Hefe ermöglichen,
- einen selektierbaren Marker für die transformierte Hefe,
- und gegebenenfalls einen Replikationsursprung und einen selektierbaren Marker für Escherichia coli,
in einem Wachstumsmedium kultiviert wird.

2. Verfahren wie in Anspruch 1 beansprucht, wobei die Expressionskassette und der selektierbare Marker in die EcoRI-Stelle von pkD1 insertiert sind.

3. Verfahren wie in einem der Ansprüche 1 und 2 beansprucht, wobei die Expressionskassette und der selektierbare Marker in eine Region aus 197 Nukleotiden insertiert sind, die definiert werden durch die SacI- und MstII-Stellen von pKD1 oder die Sphl-Stelle von pKD1.

4. Verfahren wie in einem der Ansprüche 1 bis 3 beansprucht, wobei die die Expression des strukturellen Gens ermöglichenden Sequenzen ausgewählt werden aus den Promotoren, die von Genen aus Hefen der Gattung Saccharomyces oder Kluyveromyces erhalten werden.

5. Verfahren wie in Anspruch 4 beansprucht, wobei diese Promotoren erhalten werden aus den glykolytischen Genen von Hefen der Gattung Saccharomyces oder Kluyyeromyces.

6. Verfahren wie in Anspruch 4 beansprucht, wobei die die Expression des strukturellen Gens ermöglichenden Sequenzen ausgewählt werden aus den Genen, die für Phosphoglyceratkinase (PKG), Glyceraldehyd-3-phosphat-dehydrogenase (GPD), Enolasen (ENO), Alkoholdehydrogenasen (ADH), Lactase (LAC4) oder saure Phosphatase (PHOS) kodieren.

7. Verfahren wie in Anspruch 4 beansprucht, wobei die die Expression des strukturellen Gens ermöglichenden Sequenzen ausgewählt werden aus den Genen, die für Phosphoglyceratkinase (PKG) oder saure Phosphatase (PHO5) kodieren.

8. Verfahren wie in einem der Ansprüche 1 bis 7 beansprucht, wobei den für Humanserumalbumin kodierenden Sequenzen eine Protein-Export-Sequenz vorangeht, die ausgewählt wird aus dem natürlichen N-terminalen Leader von Humanserumalbumin.

9. Verfahren wie in einem der Ansprüche 1 bis 8 beansprucht, wobei der für Humanserumalbumin kodierenden Sequenz eine Exportsequenz vorangeht, die von der natürlichen Sequenz verschieden ist.

10. Verfahren wie in Anspruch 9 beansprucht, wobei die für das Exportsignal von Humanserumalbumin kodierende Sequenz die Sequenz ist, die aus Hefegenen erhalten wird, welche für das Alphapheromon oder Killertoxin kodieren.

11. Verfahren wie in einem der Ansprüche 1 bis 10 beansprucht, wobei die die Exkretion von Humanserumalbumin ermöglichende Sequenz der natürliche präpro-terminale Leader von Albumin ist.

12. Verfahren wie in einem der Ansprüche 1 bis 11 beansprucht, wobei die selektierbaren Marker für die transformierten Hefen ausgewählt werden aus den Genen, die Resistenz gegen Antihiotika oder Kupferionen verleihen.

13. Verfahren wie in Anspruch 12 beansprucht, wobei die selektierbare Sequenz ein Gen ist, das Resistenz gegen G418 verleiht.

14. Verfahren wie in Anspruch 13 beansprucht, wobei die selektierbare Sequenz eine Fusion zwischen dem ORF1-Promoter des linearen Plasmids kl aus K. lactis und der Aminoglykosid-Phosphotransferase aus dem Transposon Tn903 ist.

15. Verfahren wie in einem der Ansprüche 1 bis 14 beansprucht, wobei der selektierbare Marker ein Gen ist, das Auxotrophien komplementiert.

16. Verfahren wie in einem der Ansprüche 1 bis 15 beansprucht, wobei der Stamm zu den Arten K. marxianus, K. wickerhamii, K. waltii gehört.

17. Verfahren wie in Anspruch 16 beansprucht, wobei der Stamm zu der Art K. bulgaricus, K. marxianus var. drosophilarum, K. marxianus var. marxianus oder K. marxianus var. lactis gehöhrt.

18. Verfahren nach einem der Ansprüche 1 bis 17, wobei Hefen in glukosehaltigem Medium kultiviert werden, zu dem Laktose zwischen der Mitte der logarithmischen (mid-log) und der frühen stationären Wachstumsphase zugegeben wird.

## Revendications

1. Procédé de préparation de sérumalbumine humaine (HSA), dans lequel on cultive dans un milieu de croissance une levure du genre Kluyveromyces transformée par un plasmide d'expression comprenant :
- les gènes fonctionnels A, B et C du plasmide pKD1,
- les unités de répétition inversées du plasmide pKD1,
- le locus de stabilité du plasmide pKD1,
- l'origine de réplication de pKD1 et une cassette d'expression contenant un ADN non-levure codant pour le gène de structure de la sérumalbumine humaine sous le contrôle de séquences permettant son expression dans ladite levure,
- un marqueur sélectionnable pour la levure transformée,
- et, éventuellement, une origine de réplication et un marqueur sélectionnable pour Escherichia coli.

2. Procédé selon la revendication 1, dans lequel la cassette d'expression et le marqueur sélectionnable sont insérés dans le site EcoRI de pKD1.

3. Procédé selon l'une quelconque des revendications 1 et 2, dans lequel la cassette d'expression et le marqueur sélectionnable sont insérés dans une région de 197 nucléotides définie par les sites Sac1 et MstII de pKD1, ou par le site SphI de pKD1.

4. Procédé selon l'une des revendications 1 à 3, dans lequel les séquences permettant l'expression du gène de structure sont choisies parmi les promoteurs dérivés des gènes de levures du genre Saccharomyces ou Kluyveromyces.

5. Procédé selon la revendication 4, dans lequel ces promoteurs dérivent des gènes glycolytiques de levures du genre Saccharomyces cerevisiae ou Kluyveromyces.

6. Procédé selon la revendication 4, dans lequel les séquences permettant l'expression du gène de structure sont choisies parmi les gènes codant pour la phosphoglycératekinase (PGK), la glycéraldéhyde-3-phosphate-déshydrogénase (GPD), les énolases (ENO), les alcool-déshydrogénases (ADH), la lactase (LAC4) ou la phosphatase acide (PHO5).

7. Procédé selon la revendication 4, dans lequel les séquences permettant l'expression du gène de structure sont choisies parmi les gènes codant pour la phosphoglycératekinase (PGK) ou la phosphatase acide (PHO5).

8. Procédé selon l'une des revendications 1 à 7, dans lequel les séquences codant pour la sérumalbumine humaine sont précédées d'une séquence d'exportation de protéines, qui est choisie dans la séquence de tête N-terminale naturelle de la sérumalbumine humaine.

9. Procédé selon l'une des revendications 1 à 8, dans lequel la séquence codant pour la sérumalbumine humaine est précédée d'une séquence d'exportation différente de la séquence naturelle.

10. Procédé selon la revendication 9, dans lequel la séquence codant pour le signal d'exportation de la sérumalbumine humaine est la séquence obtenue à partir de gènes de levures codant pour la phéromone alpha ou la toxine killer.

11. Procédé selon l'une des revendications 1 à 10, dans lequel la séquence permettant l'excrétion de la sérumalbumine humaine est la séquence de tête terminale naturelle prépro de l'albumine.

12. Procédé selon l'une des revendications 1 à 11, dans lequel les marqueurs sélectionnables pour les levures transformées sont choisis parmi les gènes assurant la résistance aux antibiotiques et aux ions cuivre.

13. Procédé selon la revendication 12, dans lequel la séquence sélectionnable est un gène assurant la résistance à G418.

14. Procédé selon la revendication 13, dans lequel la séquence sélectionnable est un produit de fusion entre le promoteur ORF1 du plasmide linéaire kl de K. lactis et l'aminoglycoside-phosphotransférase du transposon Tn903.

15. Procédé selon l'une quelconque des revendications 1 à 14, dans lequel le marqueur sélectionnable est un gène qui complète les oxotrophies.

16. Procédé selon l'une quelconque des revendications 1 à 15, dans lequel la souche est de l'espèce K. marxianus, K. wickerhamii, K. waltii.

17. Procédé selon la revendication 16, dans lequel la souche est de l'espèce K. bulgaricus, K. marxianus var. drosophilarum, K. marxianus var. marxianus ou K. marxianus var. lactis.

18. Procédé selon l'une quelconque des revendications 1 à 17, dans lequel les levures sont cultivées dans un milieu contenant du glucose, auquel du lactose est ajouté entre la phase mi-logarithmique et la phase de croissance stationnaire précoce.
